# EUROPEAN PATENT APPLICATION

(11) **EP 4 209 174 A1**
(43) Date of publication of application: **12.07.2023**
(21) Application number: 21881782.3
(22) Date of filing: 15.09.2021
(51) Int. Cl.: A61B 5/1455, A61B 5/024, A61B 5/00, G05B 19/042

(54) **METHOD FOR PERIODICALLY MEASURING BLOOD OXYGEN, AND ELECTRONIC DEVICE**

(30) Priority: 21.10.2020 CN 202011133857
(71) Applicant: Huawei Technologies Co., Ltd., Shenzhen, Guangdong 518129 (CN)
(72) Inventor: LI, Jing, Shenzhen, Guangdong 518129 (CN); WANG, Kun, Shenzhen, Guangdong 518129 (CN); LI, Weinan, Shenzhen, Guangdong 518129 (CN); LIN, Zhongwei, Shenzhen, Guangdong 518129 (CN); YE, Jilong, Shenzhen, Guangdong 518129 (CN)
(74) Representative: Isarpatent
(86) International application number: PCT/CN2021/118373
(87) International publication number: WO 2022/083363

(57) **Abstract**

A method for periodically measuring blood oxygen saturation is disclosed, and applied to an electronic device. The electronic device includes a red light R path and an infrared light IR path, and the R path and the IR path each include a light emitting source and a photoelectric detector. The method includes: The electronic device receives a first instruction indicating to periodically measure blood oxygen saturation; performs, in response to the first instruction, dimming in a first blood oxygen measurement period if a user changes from a non-sleep state to a sleep state, where the dimming is used to determine that a light emitting power of the light emitting source of the R path and a light emitting power of the light emitting source of the IR path are respectively a first power and a second power; and during blood oxygen measurement time in the first blood oxygen measurement period or blood oxygen measurement time in a next blood oxygen measurement period, if the user is in the sleep state, controls the light emitting source of the R path to emit the red light at the first power and the light emitting source of the IR path to emit the infrared light at the second power, collects a red light PPG signal of the R path and an infrared light PPG signal of the IR path, to calculate the SpO2. In the method for periodically measuring blood oxygen saturation, dimming is performed only once when the user is in the sleep state, and not performed in a sleep process, so that power consumption of the electronic device can be reduced.

## Description

This application claims priority to Chinese Patent Application No. 202011133857.2, filed with the China National Intellectual Property Administration on October 21, 2020 and entitled "METHOD FOR PERIODICALLY MEASURING BLOOD OXYGEN AND ELECTRONIC DEVICE", which is incorporated herein by reference in its entirety.

### TECHNICAL FIELD

The present invention relates to the field of computer technologies, and in particular, to a method for periodically measuring blood oxygen and an electronic device.

### BACKGROUND

Blood oxygen saturation (blood oxygen saturation, SpO2) is a percentage of oxygen-bound oxyhemoglobin (HbO2) in the blood to total hemoglobin that can be bound, that is, a concentration of oxygen in the blood, and is also a percentage of blood oxygen content to blood oxygen capacity. The blood oxygen saturation can reflect a capability of binding hemoglobin and oxygen, and is an important physiological parameter of respiratory circulation.

Generally, the SpO2 of a person should not be less than 94%. If the SpO2 is less than 94%, oxygen supply is insufficient, that is, oxygen deficiency occurs. The oxygen deficiency indicates an imbalance between the oxygen supply and oxygen consumption, and metabolism of tissue cells is in a state of hypoxia. The oxygen deficiency of a body causes many harms, and may impose a great impact on a central nervous system, liver, kidney, and another function. Short-term hypoxia may lead to a symptom such as distraction, a memory loss, dizziness, or anxiety. Severe or long-term hypoxia may lead to a myocardial failure, a blood pressure drop, a blood circulation failure, and even brain tissue degeneration and necrosis. Therefore, periodic blood oxygen measurement is very important for a user to learn of a blood oxygen status of the user. Particularly, the blood oxygen at night may enable the user to learn of a sleep breathing problem of the user, so that the user can detect and resolve the problem in time.

However, dimming needs to be performed before each blood oxygen measurement, increasing power consumption of an electronic device.

### SUMMARY

Embodiments of this application provide a method for periodically measuring blood oxygen and an electronic device. Therefore, dimming may be performed only once when a user is in a sleep state, and not be performed in a sleep process, so that power consumption of the electronic device can be reduced.

According to a first aspect, an embodiment of this application provides a method for periodically measuring blood oxygen, applied to an electronic device. The electronic device includes a red light R path and an infrared light IR path, the R path and the IR path each include a light emitting source and a photoelectric detector, the light emitting source of the R path is configured to emit red light to a user, the photoelectric detector of the R path is configured to convert red light reflected by the user into a red light photoplethysmography PPG signal, the light emitting source of the IR path is configured to emit infrared light to the user, and the photoelectric detector of the IR path is configured to convert infrared light reflected by the user into an infrared light photoplethysmography PPG signal.

The method includes:
The electronic device receives a first instruction, and the first instruction indicates to periodically measure blood oxygen saturation.

The electronic device performs, in response to the first instruction, dimming in a first blood oxygen measurement period if the user changes from a non-sleep state to a sleep state, and the dimming is used to determine that a light emitting power of the light emitting source of the R path and a light emitting power of the light emitting source of the IR path are respectively a first power and a second power

During blood oxygen measurement time in a second blood oxygen measurement period, if the user is in the sleep state, the electronic device controls the light emitting source of the R path to emit the red light at the first power and the light emitting source of the IR path to emit the infrared light at the second power. The second blood oxygen measurement period and the first blood oxygen measurement period are a same blood oxygen measurement period, or the second period is a next blood oxygen measurement period of the first blood oxygen measurement period.

The electronic device respectively collects the red light PPG signal and the infrared light PPG signal by using the photoelectric detector of the R path and the photoelectric detector of the IR path, and the red light PPG signal and the infrared light PPG signal are used to calculate the blood oxygen saturation.

In the method, dimming is performed only once when the user is in the sleep state, and not performed in a sleep process, so that power consumption of the electronic device is reduced.

In a possible implementation, the second blood oxygen measurement period and the first blood oxygen measurement period are the same blood oxygen measurement period, and the performing dimming in a first blood oxygen measurement period if the user changes from a non-sleep state to a sleep state includes:

The electronic device detects a current sleep status of the user when current time is the blood oxygen measurement time.

When the current sleep status is the sleep state and the user is in the non-sleep state in a previous blood oxygen measurement period of the first blood oxygen measurement period, the electronic device performs dimming.

In a possible implementation, the method further includes:
turning off the light emitting source of the R path, the light emitting source of the IR path, the photoelectric detector of the R path, and the photoelectric detector of the IR path during non-blood oxygen measurement time, where the non-blood oxygen measurement time is a time period other than the blood oxygen measurement time in the blood oxygen measurement period, to further reduce energy consumption of the electronic device.

In a possible implementation, before the controlling the light emitting source of the R path to emit the red light at the first power and the light emitting source of the IR path to emit the infrared light at the second power, the method further includes:

The electronic device receives a second instruction, and the second instruction indicates to continuously obtain a PPG signal.

In a possible implementation, the electronic device further includes a PPG manager, and the PPG manager of the electronic device is configured to perform the following steps:
generating a control signal based on the first instruction and the second instruction, where the first instruction comes from a first application, the second instruction comes from a second application, and the control signal is used to drive the light emitting source of the R path to emit the red light at the first power and the light emitting source of the IR path to emit the infrared light at the second power; and
sending the red light PPG signal and the infrared light PPG signal to the first application, and sending, to the second application, the PPG signal obtained based on the indication of the second instruction.

In the method, the R path and the IR path of the electronic device can be simultaneously invoked by a plurality of applications, to simultaneously measure the blood oxygen saturation, a heart rate, and the like.

In a possible implementation, when the PPG signal obtained based on the indication of the second instruction is the red light PPG signal, the method further includes:
during non-blood oxygen measurement time, keeping controlling the light emitting source of the R path to emit the red light at the first power and keeping collecting the red light PPG signal by using the photoelectric detector of the R path, where the non-blood oxygen measurement time is a time period other than the blood oxygen measurement time in the second blood oxygen measurement period; and
turning off the light emitting source of the IR path and the photoelectric detector of the IR path during the non-blood oxygen measurement time.

In a possible implementation, when the PPG signal obtained based on the indication of the second instruction is the infrared light PPG signal, the method further includes:
during non-blood oxygen measurement time, keeping controlling the light emitting source of the IR path to emit the red light at the second power and keeping collecting the infrared light PPG signal by using the photoelectric detector of the IR path, where the non-blood oxygen measurement time is a time period other than the blood oxygen measurement time in the second blood oxygen measurement period; and
turning off the light emitting source of the R path and the photoelectric detector of the R path during the non-blood oxygen measurement time.

In a possible implementation, when the PPG signal obtained based on the indication of the second instruction is the red light PPG signal and the infrared light PPG signal, the method further includes:
during non-blood oxygen measurement time, keeping controlling the light emitting source of the R path to emit the red light at the first power, keeping controlling the light emitting source of the IR path to emit the infrared light at the second power, keeping collecting the red light PPG signal by using the photoelectric detector of the R path, and keeping collecting the infrared light PPG signal by using the photoelectric detector of the IR path, where the non-blood oxygen measurement time is a time period other than the blood oxygen measurement time in the second blood oxygen measurement period.

In a possible implementation, the method further includes:

The electronic device performs dimming when the user is in the non-sleep state during the blood oxygen measurement time, and the dimming is used to determine that the light emitting power of the light emitting source of the R path and the light emitting power of the light emitting source of the IR path are respectively the first power and the second power

In a possible implementation, that the electronic device performs dimming when the user is in the non-sleep state during the blood oxygen measurement time specifically includes:
When the user is in the non-sleep state during the blood oxygen measurement time, the electronic device obtains motion data of the user from previous dimming to current time.

When it is identified, based on the motion data, that a motion amplitude of the user from the previous dimming to the current time is less than a preset value, the electronic device adjusts the light emitting power of the light emitting source of the R path and the light emitting power of the light emitting source of the IR path to be respectively the first power and the second power

In a possible implementation, the first power is a transmit power of the light emitting source of the R path applied when an output current of the photoelectric detector of the R path is greater than or equal to a first target current; and the second power is a transmit power of the light emitting source of the IR path applied when an output current of the photoelectric detector of the IR path is greater than or equal to a second target current.

In a possible implementation, a sampling frequency of the photoelectric detector of the R path applied when the user is in the sleep state is less than a sampling frequency of the photoelectric detector of the R path applied when the user is in the non-sleep state; and a sampling frequency of the photoelectric detector of the IR path when the user is in the sleep state is less than a sampling frequency of the photoelectric detector of the IR path when the user is in the non-sleep state.

In a possible implementation, the light emitting source of the R path and the light emitting source of the IR path are a same light emitting source, and the photoelectric detector of the R path and the photoelectric detector of the IR path are a same photoelectric detector.

According to a second aspect, an embodiment of this application further provides an electronic device. The electronic device includes a photoplethysmography PPG controller, a memory, a red light R path, and an infrared light IR path. The R path and the IR path each include a light emitting source and a photoelectric detector, the light emitting source of the R path is configured to emit red light to a user, the photoelectric detector of the R path is configured to convert red light reflected by the user into a red light photoplethysmography PPG signal, the light emitting source of the IR path is configured to emit infrared light to the user, and the photoelectric detector of the IR path is configured to convert infrared light reflected by the user into an infrared light photoplethysmography PPG signal. The PPG controller runs computer instructions stored in the memory, to implement the method implemented by the electronic device in any one of the first aspect or the possible implementations of the first aspect.

According to a third aspect, an embodiment of this application further provides a computer program product including instructions. When the computer program product is run on an electronic device, the electronic device is enabled to perform the method in any one of the first aspect or the possible implementations of the first aspect.

According to a fourth aspect, an embodiment of this application further provides a computer-readable storage medium, including instructions. When the instructions are run on an electronic device, the electronic device is enabled to perform the method in any one of the first aspect or the possible implementations of the first aspect.

### BRIEF DESCRIPTION OF DRAWINGS

FIG. 1A is a schematic diagram of an architecture of a control system according to an embodiment of this application;
FIG. 1B is a schematic diagram of a structure of an electronic device according to an embodiment of this application;
FIG. 2A is a schematic diagram of a structure of another electronic device according to an embodiment of this application;
FIG. 2B is a schematic diagram of a structure of still another electronic device according to an embodiment of this application;
FIG. 3A to FIG. 3H are illustrative schematic diagrams of some user interfaces according to an embodiment of this application;
FIG. 4 is an illustrative schematic diagram of a software architecture of an electronic device according to an embodiment of this application;
FIG. 5 is a schematic flowchart of a method for periodically measuring blood oxygen according to an embodiment of this application;
FIG. 6 is a schematic flowchart of a method for periodically measuring blood oxygen according to an embodiment of this application;
FIG. 7A is a schematic flowchart of another method for periodically measuring blood oxygen according to an embodiment of this application;
FIG. 7B-1 and FIG. 7B-2 are a schematic flowchart of another method for periodically measuring blood oxygen according to an embodiment of this application; and

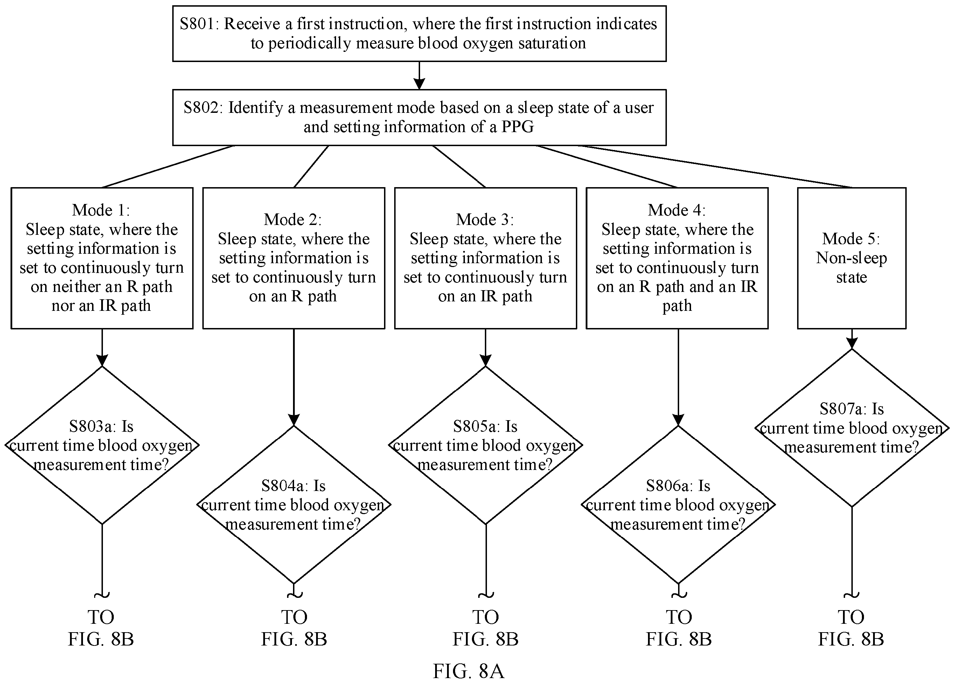

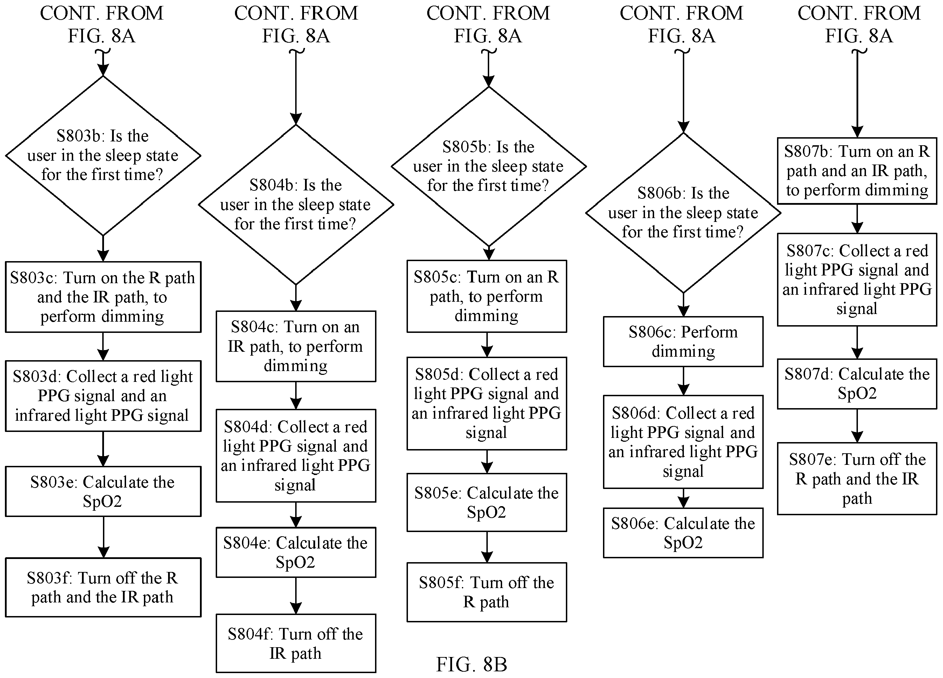

FIG. 8A and FIG. 8B are a schematic flowchart of still another method for periodically measuring blood oxygen according to an embodiment of this application.

### DESCRIPTION OF EMBODIMENTS

Terms used in the following embodiments of this application are merely intended to describe specific embodiments, but are not intended to limit embodiments of this application. Terms "one", "a", "the", "the foregoing", "this", and "the one" of singular forms used in this specification and the appended claims of this application are also intended to include plural forms, unless otherwise specified in the context clearly. It should be further understood that a term "and/or" used in embodiments of this application indicates and includes any one or all of possible combinations of one or more listed items.

The following describes concepts related to embodiments of this application.

### (1) Photoplethysmography (photoplethysmography, PPG)

PPG is a signal obtained by using a photoplethysmography method, the photoplethysmography method is a noninvasive method for detecting SpO2 based on a principle that an amount of light absorbed by arterial blood varies with a fluctuation of an artery. Specifically, oxyhemoglobin HbO2 and hemoglobin Hb have different light absorption characteristics for light with wavelengths from 600 nm to 1000 nm. An absorption coefficient of the Hb is higher when the wavelength is from 600 nm to 800 nm, and an absorption coefficient of the HbO2 is higher when the wavelength is from 800 nm to 1000 nm. Therefore, PPG signals of the HbO2 and the Hb are respectively detected by using red light (600 nm to 800 nm) and infrared light (800 nm to 1000 nm), and then a corresponding ratio is calculated through program processing. In this way, the SpO2 is obtained.

For blood oxygen measurement performed by using a wearable device, a blood oxygen value is calculated based on a ratio of a perfusion index of a red light PPG signal of a wrist to a perfusion index of an infrared light PPG signal. The perfusion index of the red light PPG signal is a ratio of a collected alternating current signal of red light to a collected direct current signal of the red light. Similarly, the perfusion index of the infrared light PPG signal is a ratio of a collected alternating current signal of infrared light to a collected direct current signal of the infrared light.

### (2) Red light (red, R) path and infrared light (infrared, IR) path

An electronic device in this application may include at least one light emitting source and at least one photoelectric detector, and may be configured to measure SpO2, a heart rate, a heart rate-related parameter, and the like. When SpO2 measurement is performed, the at least one light emitting source is configured to emit red light and infrared light, light emitted by the light emitting source is received by a photoelectric detector after being reflected, and the photoelectric detector is configured to convert the received red light into a red light PPG signal and convert the received infrared light into an infrared light PPG signal. The light emitting source may be a light emitting diode (Light emitting diode, LED), and the photoelectric detector may be a photodiode (photodiode, PD). In this embodiment of this application, the LED and the PD are used as an example for description.

It should be understood that one LED and one PD form a light path. An LED that emits red light and a PD that receives the red light form an R path, and an LED that emits infrared light and a PD that receives the infrared light form an IR path. It should be understood that the LED that emits the red light and the LED that emits the infrared light may be a same LED, and this may be implemented through time division multiplexing. Similarly, the PD that receives the red light and the PD that receives the infrared light may be a same PD.

It should be understood that when the electronic device turns on the R path, to be specific, an LED and a PD corresponding to the R path of the electronic device are turned on, the LED of the R path emits red light, and the PD of the R path receives the red light to generate a red light PPG signal. When the electronic device turns on the IR path, to be specific, an LED and a PD corresponding to the IR path of the electronic device are turned on, the LED of the IR path emits infrared light, and the PD of the IR path receives the infrared light to generate an infrared light PPG signal.

It should be further understood that turning off the R path means that the LED of the R path no longer emits the red light, and the PD of the R path no longer generates the red light PPG signal; and turning off the IR path means that the LED of the IR path no longer emits the infrared light, and the PD of the IR path no longer generates the infrared light PPG signal.

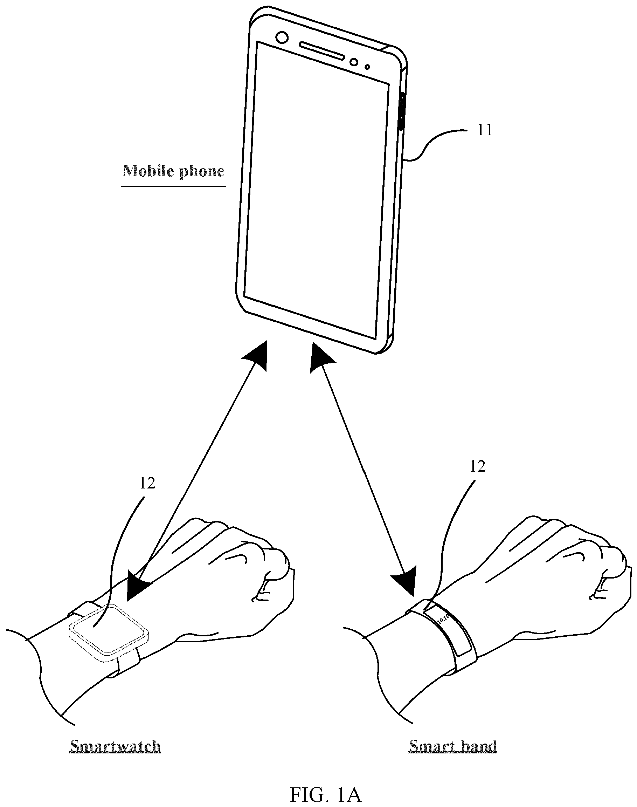

The following first describes a control system provided in an embodiment of this application. As shown in FIG. 1A, the system may include a first electronic device 11 and a second electronic device 12. The first electronic device 11 may be a wearable device that can implement blood oxygen measurement, such as a smartwatch or a smart band. The second electronic device 12 may be a device such as a mobile phone, a tablet computer, or a cloud.

The first electronic device 11 may include at least one light emitting source and at least one photoelectric detector, and may collect a PPG signal. The PPG signal may include the red light PPG signal, the infrared light PPG signal, and the like. SpO2 may be calculated based on the red light PPG signal and the infrared light PPG signal. A heart rate and a heart rate-related parameter may also be calculated based on the PPG signal.

The first electronic device 11 may periodically measure blood oxygen in response to a first instruction. The first instruction may be an instruction that is generated after the electronic device receives an input user operation and that indicates the electronic device 11 to periodically measure the blood oxygen. Alternatively, the first electronic device may receive an instruction that is sent by the second electronic device 11 and that indicates the electronic device 11 to periodically measure the blood oxygen.

The second electronic device 12 may be communicatively connected to the first electronic device 11, and send an instruction to the first electronic device 111, to control the first electronic device 11. For example, the second electronic device 12 sends the first instruction to the first electronic device 11, so that the first electronic device 11 periodically measures the SpO2 in response to the first instruction, and sends the measured SpO2 to the second electronic device 12. For another example, the second electronic device 12 sends, to the first electronic device 11, an instruction indicating to measure the heart rate, so that the first electronic device 11 performs heart rate measurement after receiving the instruction, and sends the measured heart rate to the second electronic device 12.

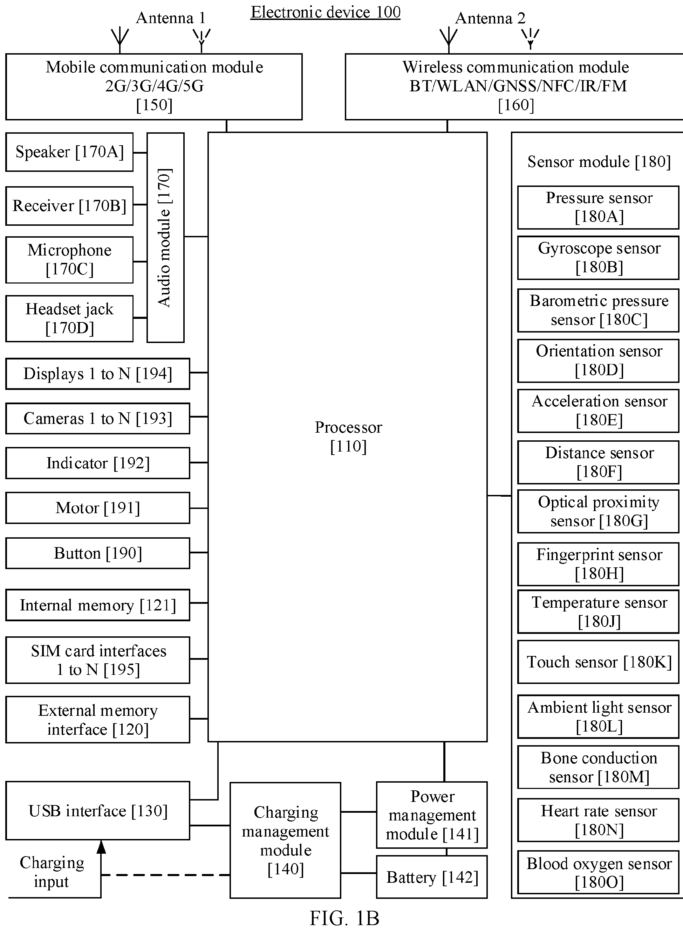

The following first describes an example electronic device provided in this embodiment of this application. The electronic device may be an electronic device that can measure SpO2, such as a smartwatch or a smart band. FIG. 1B is a schematic diagram of a structure of an electronic device according to an embodiment of this application. This electronic device 100 may be the first electronic device 11 in FIG. 1A, or may be the second electronic device 12 in FIG. 1A. The electronic device 100 may include a processor 110, an external memory interface 120, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identification module (subscriber identification module, SIM) card interface 195, and the like. The sensor module 180 may include a pressure sensor 180A, a gyroscope sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, a heart rate sensor 180N, a blood oxygen sensor 180O, and the like.

It may be understood that the structure shown in this embodiment of this application does not constitute a specific limitation on the electronic device 100. In some other embodiments of this application, the electronic device 100 may include more or fewer components than those shown in the figure, or combine some components, or split some components, or have different component arrangements. The components shown in the figure may be implemented by hardware, software, or a combination of software and hardware.

It should be noted that, when the electronic device 100 is the second electronic device 12 in FIG. 1A, the second electronic device 12 may not include the heart rate sensor 180N, the blood oxygen sensor 180O, or the like.

The processor 110 may include one or more processing units. For example, the processor 110 may include an application processor (application processor, AP), a modem processor, a graphics processing unit (graphics processing unit, GPU), an image signal processor (image signal processor, ISP), a controller, a memory, a video codec, a digital signal processor (digital signal processor, DSP), a baseband processor, a neural-network processing unit (neural-network processing unit, NPU), and/or the like. Different processing units may be independent components, or may be integrated into one or more processors.

The controller may be a nerve center and a command center of the electronic device 100. The controller may generate an operation control signal based on an instruction operation code and a time sequence signal, to complete control of instruction reading and instruction execution.

A memory may be further disposed in the processor 110, and is configured to store instructions and data. In some embodiments, the memory in the processor 110 is a cache memory. The memory may store instructions or data that have or has been used or cyclically used by the processor 110. If the processor 110 needs to use the instructions or the data again, the processor may directly invoke the instructions or the data from the memory. This avoids repeated access, reduces waiting time of the processor 110, and improves system efficiency.

In this application, the one or more processors may include a PPG controller, and may implement functions implemented by the PPG controller in this application.

In some embodiments, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

The I2C interface is a two-way synchronization serial bus, and includes a serial data line (serial data line, SDA) and a serial clock line (serial clock line, SCL). In some embodiments, the processor 110 may include a plurality of groups of I2C buses. The processor 110 may be separately coupled to the touch sensor 180K, a charger, a flash, the camera 193, and the like through different I2C bus interfaces. For example, the processor 110 may be coupled to the touch sensor 180K through the I2C interface, so that the processor 110 communicates with the touch sensor 180K through the I2C bus interface, to implement a touch function of the electronic device 100.

The I2S interface may be configured to perform audio communication. In some embodiments, the processor 110 may include a plurality of groups of I2S buses. The processor 110 may be coupled to the audio module 170 through the I2S bus, to implement communication between the processor 110 and the audio module 170. In some embodiments, the audio module 170 may transmit an audio signal to the wireless communication module 160 through the I2S interface, to implement a function of answering a call through a Bluetooth headset.

The PCM interface may also be used to perform audio communication, and sample, quantize, and code an analog signal. In some embodiments, the audio module 170 may be coupled to the wireless communication module 160 through a PCM bus interface. In some embodiments, the audio module 170 may alternatively transmit an audio signal to the wireless communication module 160 through the PCM interface, to implement a function of answering a call through a Bluetooth headset. Both the I2S interface and the PCM interface may be used for audio communication.

The UART interface is a universal serial data bus, and is used for asynchronous communication. The bus may be a two-way communication bus. The bus converts to-be-transmitted data between serial communication and parallel communication. In some embodiments, the UART interface is usually configured to connect the processor 110 to the wireless communication module 160. For example, the processor 110 communicates with a Bluetooth module in the wireless communication module 160 through the UART interface, to implement a Bluetooth function. In some embodiments, the audio module 170 may transmit an audio signal to the wireless communication module 160 through the UART interface, to implement a function of playing music through a Bluetooth headset.

The MIPI interface may be configured to connect the processor 110 to a peripheral component such as the display 194 or the camera 193. The MIPI interface includes a camera serial interface (camera serial interface, CSI), a display serial interface (display serial interface, DSI), and the like. In some embodiments, the processor 110 communicates with the camera 193 through the CSI interface, to implement a photographing function of the electronic device 100. The processor 110 communicates with the display 194 through the DSI interface, to implement a display function of the electronic device 100.

The GPIO interface may be configured by software. The GPIO interface may be configured as a control signal or a data signal. In some embodiments, the GPIO interface may be configured to connect the processor 110 to the camera 193, the display 194, the wireless communication module 160, the audio module 170, the sensor module 180, or the like. The GPIO interface may alternatively be configured as the I2C interface, the I2S interface, the UART interface, the MIPI interface, or the like.

The USB interface 130 is an interface that conforms to a USB standard specification, and may be specifically a mini USB interface, a micro USB interface, a USB type-C interface, or the like. The USB interface 130 may be configured to connect to the charger to charge the electronic device 100, or may be configured to perform data transmission between the electronic device 100 and a peripheral device, or may be configured to connect to a headset to play audio through the headset. The interface may be further configured to connect to another electronic device such as an AR device.

It may be understood that an interface connection relationship between the modules that is shown in this embodiment of the present invention is merely an example for description, and does not constitute a limitation on a structure of the electronic device 100. In some other embodiments of this application, the electronic device 100 may alternatively use an interface connection mode different from an interface connection mode in this embodiment, or a combination of a plurality of interface connection modes.

The charging management module 140 is configured to receive a charging input from the charger. The charger may be a wireless charger or a wired charger. In some embodiments of wired charging, the charging management module 140 may receive a charging input of a wired charger through the USB interface 130. In some embodiments of wireless charging, the charging management module 140 may receive a wireless charging input through a wireless charging coil of the electronic device 100. The charging management module 140 supplies power to the electronic device through the power management module 141 while charging the battery 142.

The power management module 141 is configured to connect to the battery 142, the charging management module 140, and the processor 110. The power management module 141 receives input of the battery 142 and/or the charging management module 140, to supply power to the processor 110, the internal memory 121, an external memory, the display 194, the camera 193, the wireless communication module 160, and the like. The power management module 141 may further be configured to monitor parameters such as a battery capacity, a battery cycle count, and a battery health status (electric leakage or impedance). In some other embodiments, the power management module 141 may alternatively be disposed in the processor 110. In some other embodiments, the power management module 141 and the charging management module 140 may alternatively be disposed in a same component.

A wireless communication function of the electronic device 100 may be implemented through the antenna 1, the antenna 2, the mobile communication module 150, the wireless communication module 160, the modem processor, the baseband processor, and the like.

The antenna 1 and the antenna 2 are configured to transmit and receive an electromagnetic wave signal. Each antenna in the electronic device 100 may be configured to cover one or more communication frequency bands. Different antennas may be further multiplexed, to improve antenna utilization. For example, the antenna 1 may be multiplexed as a diversity antenna of a wireless local area network. In some other embodiments, the antenna may be used in combination with a tuning switch.

The mobile communication module 150 may provide a wireless communication solution that is applied to the electronic device 100 and that includes 2G/3G/4G/5G. The mobile communication module 150 may include at least one filter, a switch, a power amplifier, a low noise amplifier (low noise amplifier, LNA), and the like. The mobile communication module 150 may receive an electromagnetic wave through the antenna 1, perform processing such as filtering or amplification on the received electromagnetic wave, and transmit the electromagnetic wave to the modem processor for demodulation. The mobile communication module 150 may further amplify a signal modulated by the modem processor, and convert the signal into an electromagnetic wave for radiation through the antenna 1. In some embodiments, at least some functional modules in the mobile communication module 150 may be disposed in the processor 110. In some embodiments, at least some functional modules of the mobile communication module 150 may be disposed in a same device as at least some modules of the processor 110.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal. Then, the demodulator transmits the low-frequency baseband signal obtained through demodulation to the baseband processor for processing. The low-frequency baseband signal is processed by the baseband processor and then transmitted to the application processor. The application processor outputs a sound signal by an audio device (which is not limited to the speaker 170A, the receiver 170B, or the like), or displays an image or a video on the display 194. In some embodiments, the modem processor may be an independent component. In some other embodiments, the modem processor may be independent of the processor 110, and is disposed in a same device as the mobile communication module 150 or another functional module.

The wireless communication module 160 may provide a wireless communication solution that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like and that is applied to the electronic device 100. The wireless communication module 160 may be one or more components integrating at least one communication processor module. The wireless communication module 160 receives an electromagnetic wave through the antenna 2, performs frequency modulation and filtering processing on an electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation through the antenna 2.

In some embodiments, in the electronic device 100, the antenna 1 and the mobile communication module 150 are coupled, and the antenna 2 and the wireless communication module 160 are coupled, so that the electronic device 100 can communicate with a network and another device by using a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), BT, a GNSS, a WLAN, NFC, FM, an IR technology, and/or the like. The GNSS may include a global positioning system (global positioning system, GPS), a global navigation satellite system (global navigation satellite system, GLONASS), a BeiDou navigation satellite system (BeiDou navigation satellite system, BDS), a quasi-zenith satellite system (quasi-zenith satellite system, QZSS), and/or a satellite based augmentation system (satellite based augmentation systems, SBAS).

The electronic device 100 may implement a display function through the GPU, the display 194, the application processor, and the like. The GPU is a microprocessor for image processing, and is connected to the display 194 and the application processor. The GPU is configured to: perform mathematical and geometric calculation, and render an image. The processor 110 may include one or more GPUs, which execute program instructions to generate or change display information.

The display 194 is configured to display an image, a video, and the like. The display 194 includes a display panel. The display panel may be a liquid crystal display (liquid crystal display, LCD), an organic light-emitting diode (organic light-emitting diode, OLED), an active-matrix organic light emitting diode (active-matrix organic light emitting diode, AMOLED), a flexible light-emitting diode (flexible light-emitting diode, FLED), a mini-LED, a micro-LED, a micro-OLED, a quantum dot light emitting diode (quantum dot light emitting diode, QLED), or the like. In some embodiments, the electronic device 100 may include one or N displays 194, where N is a positive integer greater than 1.

The electronic device 100 may implement a capture function by using the ISP, the camera 193, the video codec, the GPU, the display 194, the application processor, and the like, to implement an image capture module at an HAL layer in this embodiment of this application.

The ISP is configured to process data fed back by the camera 193. For example, during photographing, a shutter is pressed, and light is transmitted to a photosensitive element of a camera through a lens. An optical signal is converted into an electrical signal, and the photosensitive element of the camera transmits the electrical signal to the ISP for processing, to convert the electrical signal into a visible image or video. The ISP may further perform algorithm optimization on noise, brightness, and complexion of the image. The ISP may further optimize parameters such as exposure and a color temperature of a photographing scenario. In some embodiments, the ISP may be disposed in the camera 193.

The camera 193 is configured to capture a static image or a video. An optical image of an object is generated through the lens, and is projected onto the photosensitive element. The photosensitive element may be a charge coupled device (charge coupled device, CCD) or a complementary metal-oxide-semiconductor (complementary metal-oxide-semiconductor, CMOS) phototransistor. The photosensitive element converts an optical signal into an electrical signal, and then transmits the electrical signal to the ISP to convert the electrical signal into a digital image or a video signal. The ISP outputs the digital image or the video signal to the DSP for processing. The DSP converts the digital image or the video signal into an image signal or a video signal in a standard format such as RGB or YUV In some embodiments, the electronic device 100 may include one or N cameras 193, where N is a positive integer greater than 1.

The digital signal processor is configured to process a digital signal, and may process another digital signal in addition to the digital image or the video signal. For example, when the electronic device 100 selects a frequency, the digital signal processor is configured to perform Fourier transformation on frequency energy.

The video codec is configured to compress or decompress a digital video. The electronic device 100 may support one or more video codecs. In this way, the electronic device 100 may play or record videos in a plurality of coding formats, for example, moving picture experts group (moving picture experts group, MPEG)-1, MPEG-2, MPEG-3, and MPEG-4.

The NPU is a neural-network (neural-network, NN) computing processor, quickly processes input information by referring to a structure of a biological neural network, for example, by referring to a mode of transmission between human brain neurons, and may further continuously perform self-learning. Applications such as intelligent cognition of the electronic device 100 may be implemented through the NPU, for example, image recognition, facial recognition, speech recognition, and text understanding.

The external memory interface 120 may be used to connect to an external storage card, for example, a Micro SD card, to extend a storage capability of the electronic device 100. The external storage card communicates with the processor 110 through the external memory interface 120, to implement a data storage function. For example, files such as music and videos are stored in the external storage card.

The internal memory 121 may be configured to store computer-executable program code. The executable program code includes instructions. The processor 110 executes various function applications and data processing of the electronic device 100 by running the instructions stored in the internal memory 121. The internal memory 121 may include a program storage area and a data storage area. The program storage region may store an operating system, an application required by at least one function (for example, a sound playing function or an image or video playing function), and the like. The data storage area may store data (such as audio data and a phone book) created when the electronic device 100 is used, and the like. In addition, the internal memory 121 may include a high-speed random access memory, or may include a nonvolatile memory, for example, at least one magnetic disk storage device, a flash memory, or a universal flash storage (universal flash storage, UFS).

The electronic device 100 may implement an audio function, for example, music playing and recording, through the audio module 170, the speaker 170A, the receiver 170B, the microphone 170C, the headset jack 170D, the application processor, and the like.

The audio module 170 is configured to convert digital audio information into an analog audio signal for output, and is also configured to convert analog audio input into a digital audio signal. The audio module 170 may be further configured to encode and decode an audio signal. In some embodiments, the audio module 170 may be disposed in the processor 110, or some functional modules in the audio module 170 are disposed in the processor 110.

The speaker 170A, also referred to as a "loudspeaker", is configured to convert an audio electrical signal into a sound signal. The electronic device 100 may be used to listen to music or answer a call in a hands-free mode over the speaker 170A.

The receiver 170B, also referred to as an "earpiece", is configured to convert an electrical audio signal into a sound signal. When a call is answered or speech information is received through the electronic device 100, the receiver 170B may be put close to a human ear to listen to a voice.

The microphone 170C, also referred to as a "mike" or a "mic", is configured to convert a sound signal into an electrical signal. When making a call or sending a voice message, a user may make a sound near the microphone 170C through the mouth of the user, to input a sound signal to the microphone 170C. At least one microphone 170C may be disposed in the electronic device 100. In some other embodiments, two microphones 170C may be disposed in the electronic device 100, to collect a sound signal and implement a noise reduction function. In some other embodiments, three, four, or more microphones 170C may alternatively be disposed in the electronic device 100, to collect a sound signal, implement noise reduction, and identify a sound source, so as to implement a directional recording function and the like.

The headset jack 170D is configured to connect to a wired headset. The headset jack 170D may be the USB interface 130, or may be a 3.5 mm open mobile terminal platform (open mobile terminal platform, OMTP) standard interface or cellular telecommunications industry association of the USA (cellular telecommunications industry association of the USA, CTIA) standard interface.

The pressure sensor 180A is configured to sense a pressure signal, and can convert the pressure signal into an electrical signal. In some embodiments, the pressure sensor 180A may be disposed on the display 194. There are a plurality of types of pressure sensors 180A, such as a resistive pressure sensor, an inductive pressure sensor, and a capacitive pressure sensor. The capacitive pressure sensor may include at least two parallel plates made of conductive materials. When a force is applied to the pressure sensor 180A, capacitance between electrodes changes. The electronic device 100 determines pressure intensity based on the change in the capacitance. When a touch operation is performed on the display 194, the electronic device 100 detects intensity of the touch operation through the pressure sensor 180A. The electronic device 100 may also calculate a touch location based on a detection signal of the pressure sensor 180A. In some embodiments, touch operations that are performed in a same touch position but have different touch operation intensity may correspond to different operation instructions. For example, when a touch operation whose touch operation intensity is less than a first pressure threshold is performed on an SMS message application icon, an instruction for viewing an SMS message is performed. When a touch operation whose touch operation intensity is greater than or equal to the first pressure threshold is performed on the SMS message application icon, an instruction for creating a new SMS message is performed.

The gyroscope sensor 180B may be configured to determine a moving posture of the electronic device 100. In some embodiments, an angular velocity of the electronic device 100 around three axes (namely, x, y, and z axes) may be determined by using the gyroscope sensor 180B. The gyroscope sensor 180B may be configured to implement image stabilization during photographing. For example, when the shutter is pressed, the gyroscope sensor 180B detects an angle at which the electronic device 100 jitters, calculates, based on the angle, a distance for which a lens module needs to compensate, and allows the lens to cancel the jitter of the electronic device 100 through reverse motion, to implement image stabilization. The gyroscope sensor 180B may also be used in a navigation scenario and a somatic game scenario.

The barometric pressure sensor 180C is configured to measure barometric pressure. In some embodiments, the electronic device 100 calculates an altitude through the barometric pressure measured by the barometric pressure sensor 180C, to assist in positioning and navigation.

An orientation sensor 180D may be a compass and/or a magnetometer, and is configured to measure included angles between the electronic device 100 and four directions: east, south, west, and north, to position the electronic device 100.

The acceleration sensor 180E may detect accelerations in various directions (usually on three axes) of the electronic device 100, and when the electronic device 100 is static, a magnitude and a direction of gravity may be detected. The acceleration sensor 180E may be further configured to identify a posture of the electronic device, and is used in an application such as switching between a landscape mode and a portrait mode or a pedometer.

The distance sensor 180F is configured to measure a distance. The electronic device 100 may measure the distance in an infrared manner or a laser manner. In some embodiments, in a photographing scenario, the electronic device 100 may measure a distance through the distance sensor 180F to implement quick focusing.

The optical proximity sensor 180G may include, for example, a light-emitting diode (LED), and an optical detector, for example, a photodiode. The light emitting diode may be an infrared light emitting diode. The electronic device 100 emits infrared light by using the light-emitting diode. The electronic device 100 detects infrared reflected light from a nearby object through the photodiode. When sufficient reflected light is detected, it may be determined that there is an object near the electronic device 100. When insufficient reflected light is detected, the electronic device 100 may determine that there is no object near the electronic device 100. The electronic device 100 may detect, by using the optical proximity sensor 180G, that the user holds the electronic device 100 close to an ear for a call, to automatically perform screen-off for power saving.

The ambient light sensor 180L is configured to sense ambient light brightness. The electronic device 100 may adaptively adjust brightness of the display 194 based on the sensed ambient light brightness. The ambient light sensor 180L may also be configured to automatically adjust white balance during photographing.

The fingerprint sensor 180H is configured to collect a fingerprint. The electronic device 100 may use a feature of the collected fingerprint to implement fingerprint-based unlocking, application lock access, fingerprint-based photographing, fingerprint-based call answering, and the like.

The temperature sensor 180J is configured to detect a temperature. In some embodiments, the electronic device 100 executes a temperature processing policy based on the temperature detected by the temperature sensor 180J. For example, when the temperature reported by the temperature sensor 180J exceeds a threshold, the electronic device 100 lowers performance of a processor nearby the temperature sensor 180J, to reduce power consumption for thermal protection. In some other embodiments, when the temperature is less than another threshold, the electronic device 100 heats the battery 142 to prevent the electronic device 100 from being shut down abnormally due to a low temperature. In some other embodiments, when the temperature is lower than still another threshold, the electronic device 100 boosts an output voltage of the battery 142 to avoid abnormal shutdown caused by a low temperature. In still some embodiments, the electronic device 100 may be a smartwatch or a smart band, and the temperature sensor 180J may be disposed on a surface that is of the electronic device 100 and that is in contact with skin, to measure a skin temperature of the user.

The touch sensor 180K is also referred to as a "touch panel". The touch sensor 180K may be disposed on the display 194, and the touch sensor 180K and the display 194 form a touchscreen, which is also referred to as a "touch screen". The touch sensor 180K is configured to detect a touch operation performed on or near the touch sensor. The touch sensor may transfer the detected touch operation to the application processor to determine a type of the touch event. A visual output related to the touch operation may be provided through the display 194. In some other embodiments, the touch sensor 180K may alternatively be disposed on a surface of the electronic device 100 at a location different from that of the display 194.

The bone conduction sensor 180M may obtain a vibration signal. In some embodiments, the bone conduction sensor 180M may obtain a vibration signal of a vibration bone of a human vocal-cord part. The bone conduction sensor 180M may also be in contact with a body pulse to receive a blood pressure beating signal. In some embodiments, the bone conduction sensor 180M may alternatively be disposed in the headset, to obtain a bone conduction headset. The audio module 170 may obtain a speech signal through parsing based on the vibration signal that is of the vibration bone of the vocal-cord part and that is obtained by the bone conduction sensor 180M, to implement a speech function. The application processor may parse heart rate information based on the blood pressure beating signal obtained by the bone conduction sensor 180M, to implement a heart rate detection function.

The heart rate sensor 180N is configured to detect a heart rate of the user. In some embodiments, the heart rate sensor may measure the heart rate and the heart rate-related parameter based on a principle of a photoplethysmography method. A measurement principle of the method is that reflection of human body tissue (such as skin, bone, meat, and fat) to light is fixed; and capillaries, arteries, veins, and the like change with the heart and pulse volumes change regularly, so that reflection to light are fluctuation values, and a frequency of this fluctuation is the heart rate. Optionally, the heart rate sensor may include a light emitting source and a photoelectric detector. The heart rate sensor detects, by using the photoelectric detector, a reflection amount of light emitted by the light emitting source, to obtain a PPG signal, and detects the heart rate-related parameter based on the PPG signal. It should be understood that light used for detecting the heart rate and the heart rate-related parameter may be red light, infrared light, green light, or the like.

The blood oxygen sensor 180O may include at least one light emitting source and at least one photoelectric detector. The at least one light emitting source may emit red light and infrared light, and the emitted red light and infrared light are reflected by the human tissue. The at least one photoelectric detector may receive the reflected light and respectively convert the reflected light into a red light PPG signal and an infrared light PPG signal. The red light PPG signal and the infrared light PPG signal are used to calculate the SpO2. For example, the blood oxygen sensor includes two LEDs and two PDs. One LED may emit red light, one LED may emit near infrared light, one PD is configured to detect the red light, and one PD is configured to detect the near infrared light.

It should be understood that the heart rate sensor 180N and the blood oxygen sensor 180O may share the light emitting source and the photoelectric detector.

The button 190 includes a power button, a volume button, and the like. The button 190 may be a mechanical button, or may be a touch button. The electronic device 100 may receive a key input, and generate a key signal input related to a user setting and function control of the electronic device 100.

The motor 191 may generate a vibration prompt. The motor 191 may be configured to provide an incoming call vibration prompt and a touch vibration feedback. For example, touch operations performed on different applications (for example, photographing and audio play) may correspond to different vibration feedback effects. The motor 191 may also correspond to different vibration feedback effects for touch operations performed on different areas of the display 194. Different application scenarios (for example, a time reminder, information receiving, an alarm clock, and a game) may also correspond to different vibration feedback effects. A touch vibration feedback effect may be further customized.

The indicator 192 may be an indicator light, and may be configured to indicate a charging status and a power change, or may be configured to indicate a message, a missed call, a notification, or the like.

The SIM card interface 195 is configured to connect to a SIM card. The SIM card may be inserted into the SIM card interface 195 or removed from the SIM card interface 195, to implement contact with or separation from the electronic device 100. The electronic device 100 may support one or N SIM card interfaces, where N is a positive integer greater than 1. The SIM card interface 195 may support a nano-SIM card, a micro-SIM card, a SIM card, and the like. A plurality of cards may be inserted into a same SIM card interface 195 at the same time. The plurality of cards may be of a same type or different types. The SIM card interface 195 may be compatible with different types of SIM cards. The SIM card interface 195 is also compatible with an external storage card. The electronic device 100 interacts with a network through the SIM card, to implement functions such as conversation and data communication. In some embodiments, the electronic device 100 uses an eSIM, that is, an embedded SIM card. The eSIM card may be embedded into the electronic device 100, and cannot be separated from the electronic device 100.

In another implementation, a PPG manager, the heart rate sensor, and the blood oxygen sensor of the electronic device may be located on a same chip.

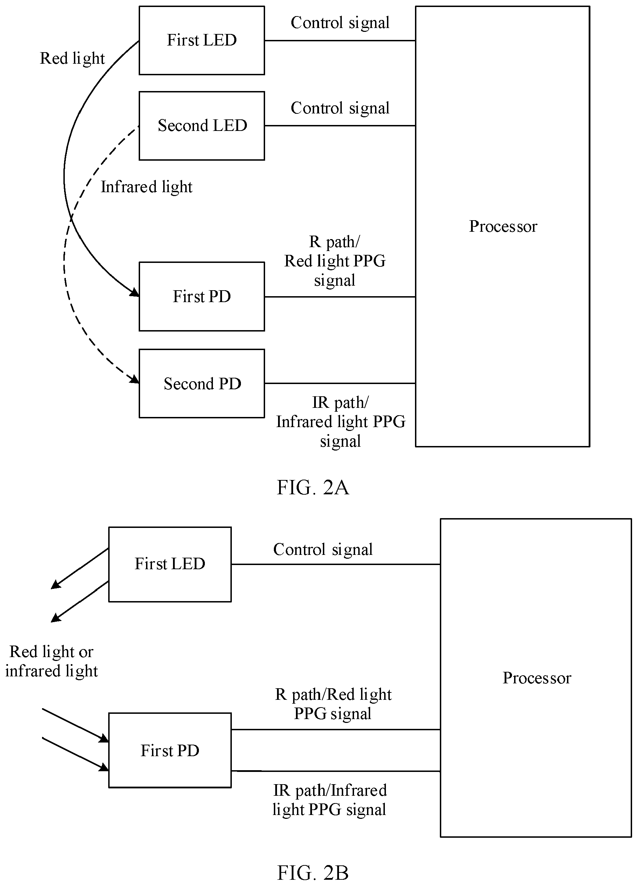

FIG. 2A is a schematic diagram of a structure of another electronic device according to an embodiment of this application. The electronic device may be the first electronic device 11 in FIG. 1A, and includes a first LED, a second LED, a first PD, a second PD, a processor, a memory (not shown in the figure), and the like. The first LED is configured to emit red light based on a received control signal sent by the processor, the red light emitted by the first LED is reflected by the first PD after being reflected, and the first PD converts the received red light into a red light PPG signal, that is, an R path. The second LED is configured to emit infrared light based on a received control signal sent by the processor, the infrared light emitted by the second LED is received by the second PD after being reflected, and the second PD converts the received infrared light into an infrared light PPG signal, that is, an IR path.

It should be understood that the electronic device may alternatively include a PD. The PD may convert received red light into a red light PPG signal, and convert received infrared light into an infrared light PPG signal. In this case, the first LED and the second LED may not emit light at the same time. The first LED and the PD form an R path, and the second LED and the PD form an IR path.

FIG. 2B is a schematic diagram of a structure of still another electronic device according to an embodiment of this application. The electronic device may be the first electronic device 11 in FIG. 1A, and includes a first LED, a first PD, a processor, a memory (not shown in the figure), and the like. The first LED and the first PD may be used in a multiplexed manner (at different sampling stages), to monitor different parameters. Specifically, after receiving a control signal sent by the processor, the first LED may emit red light and/or infrared light based on the control signal, the light emitted by the first LED is received by the first PD after being reflected, and the first PD can convert the received red light into a red light PPG signal, and convert the received infrared light into an infrared light PPG signal.

Further, SpO2, a heart rate, a heart rate-related parameter, and the like of a user are measured based on the red light PPG signal, the infrared light PPG signal, and the like. Further, a sleep status of the user may be further detected based on the measured SpO2, the heart rate, the heart rate-related parameter, and the like.

It should be understood that the first LED, the second LED, the first PD, and the second PD in FIG. 2A, and the first LED and the first PD in FIG. 2B are all disposed on a side that is of the electronic device and that is in contact with human tissue.

It should be further understood that the processors in FIG. 2A and FIG. 2B each may be a PPG controller, to implement a function implemented by the PPG controller in this application.

User interfaces in embodiments of this application are described below.

In some embodiments, a first electronic device may independently implement periodic blood oxygen measurement. Alternatively, the first electronic device may interact with a user through a user interface, to trigger the first electronic device to periodically measure SpO2.

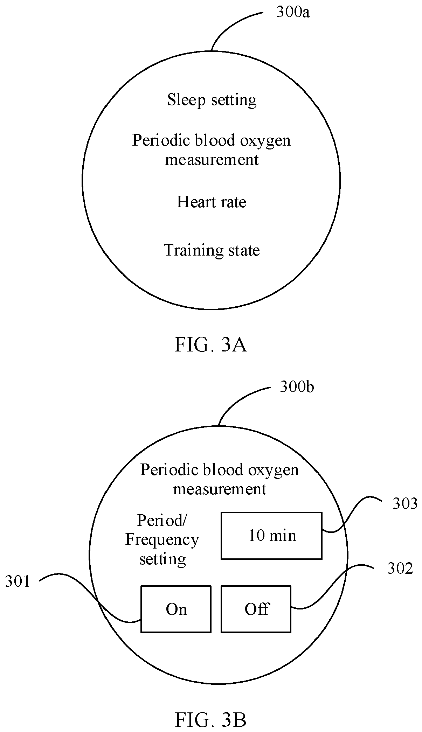

The first electronic device may display a user interface 300a shown in FIG. 3A. The user interface 300a may be a home screen, also referred to as a launcher (launcher), and includes an icon and/or an identifier of at least one application, for example, an icon and/or an identifier of Sleep setting, Periodic blood oxygen measurement, Heart rate, and Training state. In this embodiment of this application, an example in which a first application is "Periodic blood oxygen measurement" and a second application is "Sleep setting" is used for description.

The first electronic device may display a user interface 300b in FIG. 3B in response to a user operation input for an icon and/or an identifier of Periodic blood oxygen measurement. The user interface 300b may include a first control 301 and a second control 304. When receiving a user operation input for the first control 301, the first electronic device may generate and execute a first instruction in response to the user operation, and the first instruction indicates to periodically measure blood oxygen saturation. When receiving a user operation input for the second control 302, the first electronic device may generate and execute a second instruction in response to the user operation, and the second instruction indicates to stop periodically measuring the blood oxygen saturation. Optionally, the user interface 300 may further include a first input box 303. In response to a period/frequency input by the user in the first input box 303, the first electronic device periodically measures the blood oxygen saturation based on the period/frequency. For a method for periodically measuring the blood oxygen saturation by the first electronic device, refer to related descriptions in the following method embodiment. Details are not described herein again.

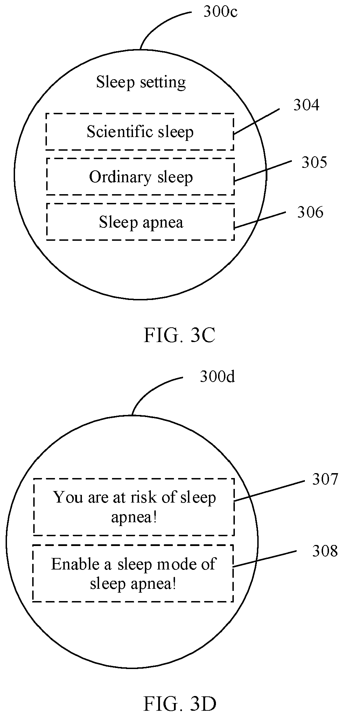

The first electronic device may display a user interface 300c in FIG. 3C in response to a user operation input for an icon and/or an identifier of "Sleep setting". The user interface 300c may include a third control 304, a fourth control 305, a fifth control 306, and the like. The third control 304 may be configured to indicate to set to a scientific sleep mode, the fourth control 305 may be configured to indicate to set to an ordinary sleep mode, and the fifth control 306 may be configured to indicate to set to a sleep apnea sleep mode.

When receiving a user operation input for the third control 304, in response to the user operation, the first electronic device may turn on an R path or an IR path, and detect a heart rate and/or a heart rate-related parameter of the user based on a PPG signal obtained through measurement on the R path or the IR path, to evaluate a sleep status of the user, for example, a deep sleep time period, deep sleep duration, light sleep duration, or a light sleep time period based on the detected heart rate and/or heart rate-related parameter.

When receiving a user operation input for the fourth control 305, in response to the user operation, the first electronic device may turn on neither an R path nor an IR path.

When receiving a user operation input for the fifth control 306, in response to the user operation, the first electronic device may turn on an R path and an IR path, and detect a heart rate and/or a heart rate-related parameter of the user based on a PPG signal obtained through measurement on the R path or the IR path, to evaluate a sleep state of the user, for example, a deep sleep time period, deep sleep duration, light sleep duration, or a light sleep time period based on the detected heart rate and/or heart rate related parameter. In addition, the first electronic device may calculate the SpO2 based on a red light PPG signal obtained through measurement on the R path and an infrared light PPG signal obtained through measurement on the IR path, and detect whether the user encounters sleep apnea based on the calculated SpO2.

Optionally, when the first electronic device detects that the SpO2 is less than a preset value (for example, 90% or 70%), it indicates that the user is at risk of sleep apnea. In this case, the first electronic device may display a user interface 300d shown in FIG. 3D, and the 300d may include first information 307 and second information 308. The first information 307 is used to prompt that the user is at risk of sleep apnea, and the second information 308 is used to prompt the user to wear a wearable device that supports sleep apnea monitoring, or to prompt the user to enable a sleep mode of sleep apnea monitoring.

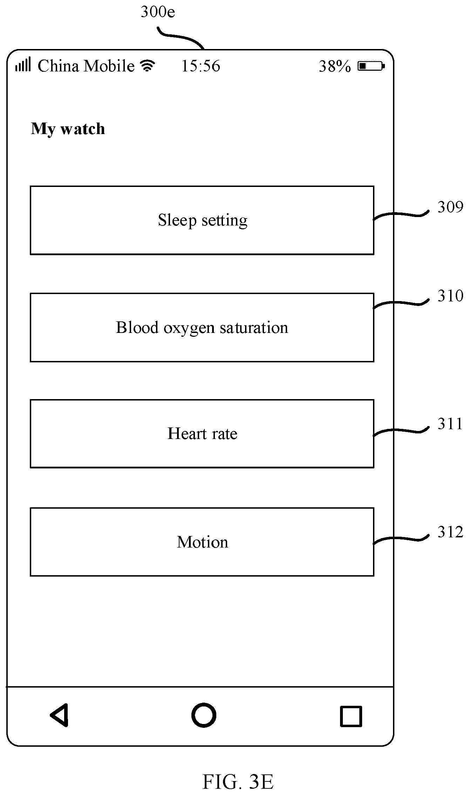

In some embodiments, a second electronic device and a first electronic device may cooperate to periodically measure blood oxygen. The second electronic device is used as a device for controlling the first electronic device. The second electronic device may run an application used to control the first electronic device 11, and display a user interface 300e shown in FIG. 3E. The user interface 300e is the user interface for controlling a first electronic device (such as a watch), and may include at least one display area, for example, a first display area 309, a second display area 310, a third display area 311, or a fourth display area 312, indicating control of at least one function that can be implemented.

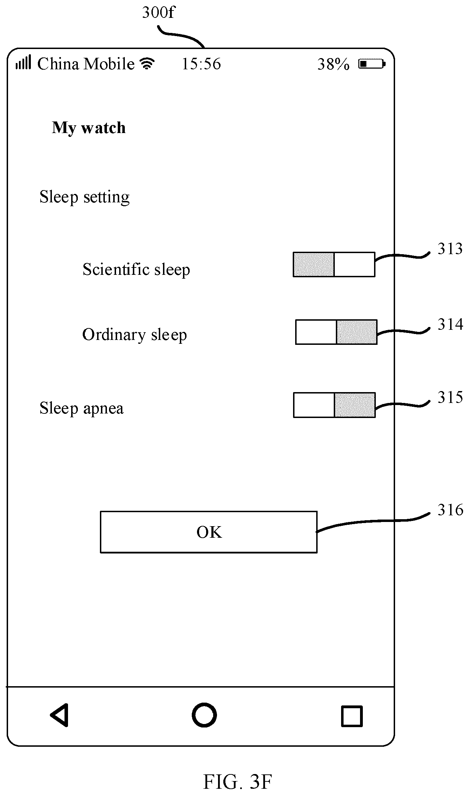

The second electronic device may enter, in response to a user operation input for the first display area 309, a setting interface of a sleep mode, for example, a user interface 300f shown in FIG. 3F. The user interface 300f may be configured to set at least one sleep mode, for example, three sleep modes such as scientific sleep, ordinary sleep, and sleep apnea, in which the first electronic device can run. The user interface 300f may include at least one control corresponding to each sleep mode, for example, a sixth control 313, a seventh control 314, and an eighth control 315, and may further include a ninth control. The second electronic device selects, in response to a user operation input for the sixth control 313, a sleep mode corresponding to the sixth control 313, that is, selects the scientific sleep. Similarly, the second electronic device selects, in response to a user operation input for the seventh control 314, a sleep mode corresponding to the seventh control 314, that is, selects the ordinary sleep. The second electronic device selects, in response to a user operation input for the eighth control 315, a sleep mode corresponding to the eighth control 315, that is, selects the sleep apnea. In response to a user operation input for the ninth control 316, the second electronic device sends, to the first electronic device, indication information indicating to enable a selected sleep mode. As shown in FIG. 3F, the second electronic device sends, to the first electronic device, the indication information indicating to enable the scientific sleep.

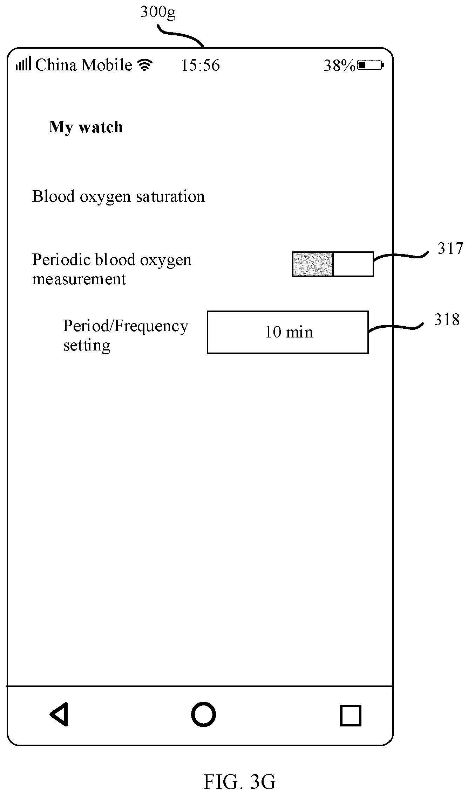

The second electronic device may enter, in response to a user operation input for the second display area 310, a setting interface of blood oxygen saturation, for example, a user interface 300g shown in FIG. 3G. The user interface 300g may include a tenth control 317 and a second input box 318. When responding to a user operation input for the tenth control 317, the second electronic device generates a first instruction and sends the first instruction to the first electronic device, and the first instruction indicates to periodically measure the blood oxygen saturation. In response to a period/frequency input by the user in the second input box 318, the second electronic device sends the input period/frequency to the first electronic device, to indicate the first electronic device to periodically measure the blood oxygen saturation based on the period/frequency. For a method for periodically measuring the blood oxygen saturation by the first electronic device, refer to related descriptions in the following method embodiment. Details are not described herein again.

Optionally, the second electronic device may enter a setting interface of Heart rate in response to a user operation input for the third display area 311, to perform heart rate detection, analysis, and the like by using the first electronic device. In response to a user operation input for the fourth display area 312, a setting interface of Motion may be displayed, to detect, by using the first electronic device, a motion state of the user. Setting interfaces of Heart rate, Motion, and the like may be implemented in a plurality of forms. This is not limited herein.

In some embodiments, after detecting the PPG signal used to measure the SpO2, the heart rate, and the like, the first electronic device may send the PPG signal to the second electronic device, and the second electronic device analyzes the PPG signal, to obtain information such as the SpO2, the heart rate, and the heart rate-related parameter. Further, the sleep status may also be analyzed based on the obtained information such as the SpO2, the heart rate, and the heart rate-related parameter.

In some other embodiments, the first electronic device may also analyze the PPG signal, to obtain information such as the SpO2, the heart rate, and the heart rate-related parameter, and send, to the second electronic device, the SpO2, the heart rate, and the heart rate-related parameter that are obtained through analysis. The second electronic device analyzes the sleep status based on the obtained information such as the SpO2, the heart rate, and the heart rate-related parameter.

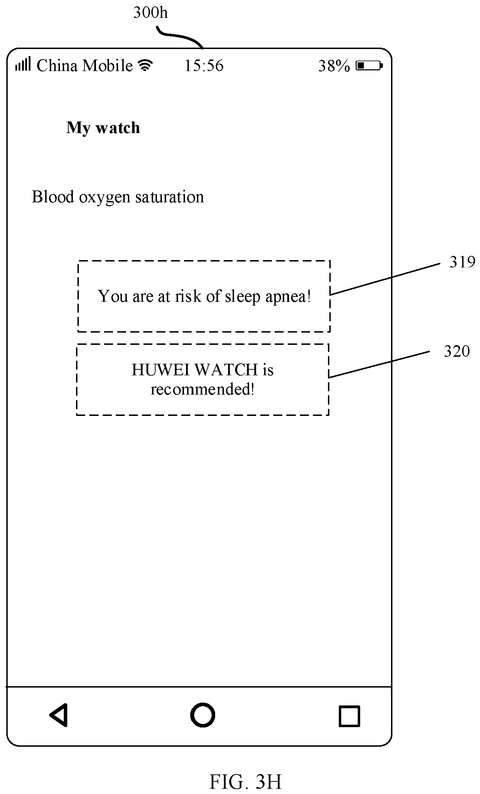

Optionally, when detecting that the SpO2 is less than a preset value (for example, 90% or 70%), the first electronic device may send, to the second electronic device, indication information indicating that the user is at risk of sleep apnea. After receiving the indication information, the second electronic device may display a user interface 300h shown in FIG. 3H. The user interface 300h may include first information 319 and second information 320. For the first information and the second information, refer to related descriptions shown in FIG. 3D. Further, in response to an input user operation for an identifier or icon of a wearable device in the second information, the second electronic device may open a purchase web page of the wearable device.

The foregoing user operation may include but is not limited to a tap operation, a double tap operation, a long press operation, or a short press operation.

In this embodiment of this application, a software system of an electronic device may use a hierarchical architecture, such as an event-driven architecture, a microkernel architecture, a microservice architecture, or a cloud architecture.

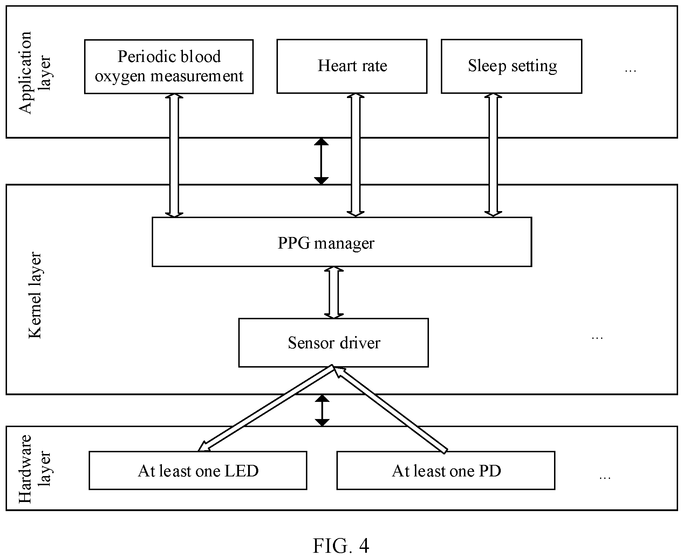

FIG. 4 is a block diagram of an example of a software structure of a first electronic device according to an embodiment of this application. In a hierarchical architecture, software is divided into several layers, and each layer has a clear role and task. The layers communicate with each other through a software interface.

As shown in FIG. 4, a software system is divided into three layers: an application layer and a kernel layer (kernel) from top to bottom.

A hardware layer may include at least one LED and at least one PD, for example, the first LED and the first PD shown in FIG. 2A, or the first LED, the second LED, the first PD, and the second PD shown in FIG. 2B. The hardware layer may further include a display and a blood oxygen sensor, configured to obtain a red light PPG signal and a near IR PPG signal.

The kernel layer is a layer between the hardware layer and a software layer. The kernel layer may include a sensor driver and a PPG manager. The sensor driver is configured to drive an LED of the hardware layer to emit light of a specific wavelength based on a received control signal. When the sensor driver detects a PPG signal, a corresponding hardware interrupt is sent to the kernel layer. The kernel layer sends the detected PPG signal to the PPG manager at the kernel layer

In addition to the foregoing driver, the kernel layer may further include a display driver, a camera driver, an audio driver, and the like.

The PPG manager is configured to obtain the PPG signal and send the PPG signal to the application layer; and is further configured to generate a control signal based on an instruction delivered by an application framework layer, and send the control signal to the sensor driver, so that the sensor driver controls, based on the control signal, the LED to emit light.

The application layer includes a series of application packages, for example, including an application "Periodic blood oxygen measurement" that can implement periodic blood oxygen measurement, an application "Heart rate" that can implement heart rate measurement, and an application " Sleep setting" that can implement sleep detection. In addition to the foregoing applications, some other applications may also be included, such as Heart rate, Exercise, Gallery, Calendar, Call, map, Navigation, WLAN, Bluetooth, Music, and Messages. In this embodiment of this application, an example in which a first application is "Periodic blood oxygen measurement" and a second application is " Sleep setting" is used for description.

As shown in FIG. 4, at the application layer, the application "Periodic blood oxygen measurement" may include an SpO2 measurement unit, the application "Heart rate" may include a heart rate measurement unit and a heart rate-related parameter measurement unit, and the application "Sleep setting" may include an SpO2 measurement unit, a heart rate measurement unit, a heart rate-related parameter measurement unit, and the like. The SpO2 measurement unit is configured to calculate SpO2 based on a red light PPG signal and an infrared light PPG signal. The heart rate measurement unit is configured to calculate a heart rate based on the PPG signal such as the red light PPG signal or the infrared light PPG signal. An SpO2 heart rate measurement unit calculates a heart rate-related parameter based on the PPG signal such as the red light PPG signal or the infrared light PPG signal.

The application may send an instruction to the PPG manager. For example, the first application "Periodic blood oxygen measurement" sends, to the PPG manager, a first instruction indicating to periodically measure the SpO2. For another example, the second application "Sleep setting" may send a second instruction to the PPG, and the second instruction may indicate a sleep mode, or may indicate a PPG signal that needs to be obtained.

After receiving the first instruction sent by the application, the PPG manager periodically measures the SpO2 in response to the first instruction. Before measuring the blood oxygen in each blood oxygen measurement period, the PPG determines, based on a sleep status of a user, whether dimming is required. When dimming is required, the PPG sends a dimming instruction to the sensor driver, so that the sensor driver drives the LED and the PD to perform dimming based on the dimming instruction, to implement dimming. When dimming is not required, a control signal indicating to turn on an R path and an IR path is sent to the sensor driver, to collect PPG data.

An implementation in which the PPG manager determines whether dimming is required may be: If it is detected that the user changes from a non-sleep state to a sleep state in a current blood oxygen measurement period, dimming is performed; if it is detected that the user is in a non-sleep state during blood oxygen measurement time of a current blood oxygen measurement period, dimming is performed; or if it is detected that the user keeps a sleep state during blood oxygen measurement time of a current blood oxygen measurement period, dimming does not need to be performed in the current blood oxygen measurement period, and the PPG data may be directly obtained during the blood oxygen measurement time.

The second instruction may have different forms. In some embodiments, the second instruction is generated by an application after a specified sleep mode is selected by the user, and the second instruction indicates to obtain a PPG signal required by the specified sleep mode.

For example, when the specified sleep mode is "scientific sleep", the application sends, to the PPG manager, an instruction S1 indicating to obtain a continuous red light PPG signal or infrared light PPG signal, and the application at the application layer may send the instruction S1 to the PPG manager. Correspondingly, the PPG manager sends, to the sensor driver based on the instruction S1, a control signal indicating to continuously turn on the R path or the IR path. The sensor driver may perform light emitting control on the LED based on the control signal, including control of light emitting time, light emitting duration, a light emitting wavelength, a light emitting power, and the like. In a process of emitting light by the LED, the sensor driver may receive the PPG signal detected by the PD, including the red light PPG signal or the infrared light PPG signal, and send the PPG signal to the PPG manager. The PPG manager may select a required PPG signal based on the control signal, to send the required PPG signal to the application at the application layer or the like.

For another example, when the specified sleep mode is "sleep apnea", the application sends, to the PPG manager, an instruction S2 indicating to obtain a continuous red light PPG signal and infrared light PPG signal. The PPG manager sends, to the sensor driver based on the instruction S2, a control signal indicating to continuously turn on the R path and the IR path.

Optionally, if the PPG manager receives the first instruction after receiving the instruction S1, the PPG manager sends, to the sensor driver, an instruction indicating a path which is not currently turned on. If the PPG manager receives the first instruction after receiving the instruction S2, the PPG manager does not need to send, to the sensor driver, the control signal indicating to continuously turn on the R path and the IR path.

Further, after collecting the PPG signal, the PPG manager may obtain, from the PPG signal based on the control signal, the red light PPG signal and the infrared light PPG signal that are required for periodically measuring blood oxygen, to obtain the PPG signal indicated by the second instruction, send, to the first application, the red light PPG signal and the infrared light PPG signal that are required for periodically measuring blood oxygen, and send, to the second application, the PPG signal corresponding to the second instruction.

In some embodiments, the software system may further include an application architecture layer, and the application architecture layer provides an application programming interface (application programming interface, API) and a programming framework for an application at the application layer. The application framework layer includes some predefined functions, for example, includes functions used to implement functions of the SpO2 measurement unit, the heart rate measurement unit, and the heart rate-related parameter measurement unit. In this case, the application at the application layer can implement measurement of the SpO2, the heart rate and the heart rate related-parameter by invoking the functions of the application architecture layer

The SpO2 measurement unit receives the red light PPG signal and the infrared light PPG signal from the PPG manager, calculates the SpO2 based on the received red light PPG signal and the received infrared light PPG signal, and reports the calculated SpO2 to the application at the application layer, such as "Periodic blood oxygen measurement" or "Sleep setting". The heart rate measurement unit receives the PPG signal from the PPG manager (may be a red light PPG signal, an infrared light PPG signal, or a green light PPG signal), calculates the heart rate based on the received PPG signal, and reports the calculated heart rate to the application at the application layer, such as "Heart rate" or " Sleep setting". The heart rate-related parameter measurement unit receives the PPG signal from the PPG manager (may be a red light PPG signal, an infrared light PPG signal, or a green light PPG signal), calculates the heart rate-related parameter based on the received PPG signal, and reports the calculated heart rate-related parameter to the application at the application layer, such as "Heart rate" or " Sleep setting".

In some embodiments, the application may invoke an application interface of the application framework layer to obtain the SpO2, the heart rate, the heart rate-related parameter, and the like.

In another implementation, in a blood oxygen measurement period, after receiving the first instruction sent by the application, the PPG manager sends, in response to the first instruction, a dimming instruction to the sensor driver when the user changes from a non-sleep state to a sleep state, so that the sensor driver perform dimming based on the dimming instruction; no dimming is performed after the user enters a sleep state; alternatively, when the user is in a non-sleep state, a dimming instruction is sent to the sensor driver when current time is blood oxygen measurement time, so that the sensor driver at the kernel layer performs dimming based on the dimming instruction.

In still another implementation, the PPG manager may determine a PPG measurement mode based on a detected status of the user (for example, a sleep state or a motion state) and setting information of the R path and the IR path, and perform dimming, control of the R path and the IR path, and the like based on the determined mode.

It should be noted that a functional architecture of the first electronic device shown in FIG. 4 is merely an implementation of this embodiment of this application. In actual application, the first electronic device may further include more or fewer software modules. This is not limited herein.

In this embodiment of this application, based on the software structure shown in FIG. 4, the electronic device may further display a corresponding user interface based on running of each software module. For a user interface displayed by the first electronic device, refer to FIG. 3A to FIG. 3D. Details are not described herein again.

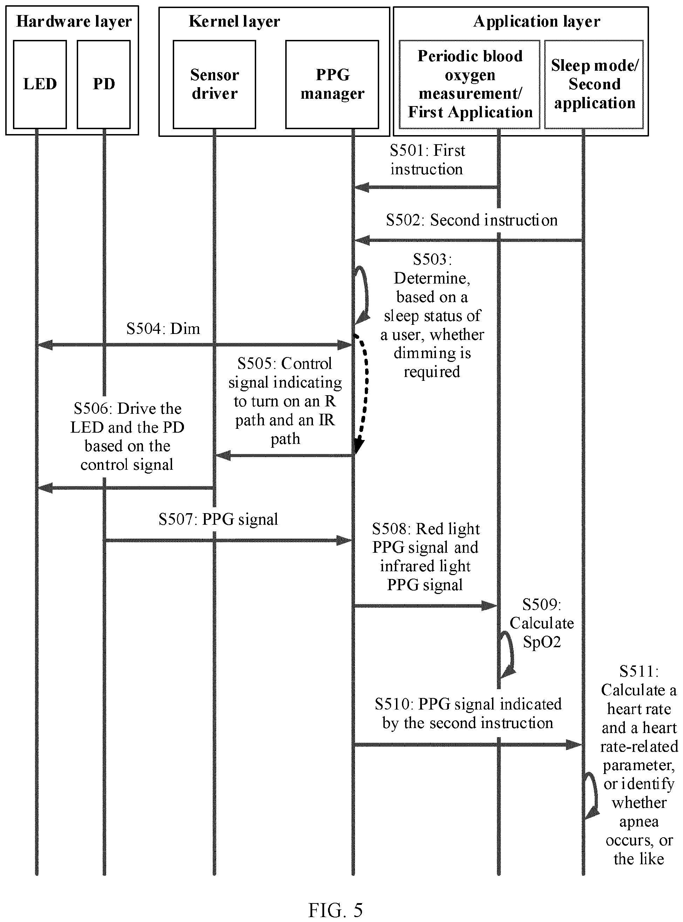

Based on the diagram of the software architecture shown in FIG. 4, the following uses a specific example to describe the method for periodically measuring blood oxygen provided in this embodiment of this application. FIG. 5 is a schematic flowchart of periodically measuring blood oxygen according to an embodiment of this application. For an application interface related to the method, refer to FIG. 3A to FIG. 3H. For implementation of steps S501 to S511 in FIG. 5, refer to related descriptions in FIG. 4. Details are not described herein again.

It should be further noted that, in some other embodiments, the first application and the second application may alternatively be two applications on the second electronic device.

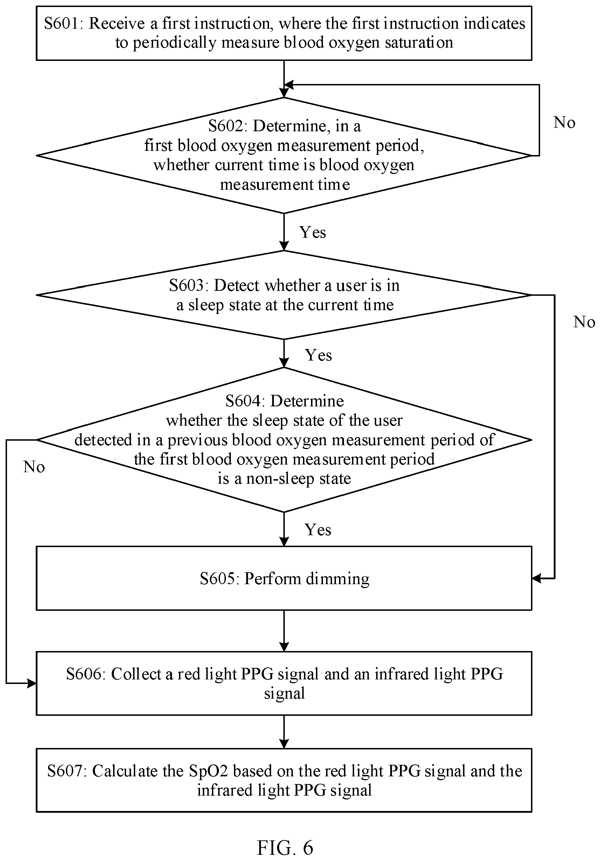

The following describes the method for periodically measuring blood oxygen in this embodiment of this application with reference to FIG. 6 to FIG. 8A and FIG. 8B.

FIG. 6 is a schematic flowchart of a method for periodically measuring blood oxygen according to an embodiment of this application. The method may be implemented by the first electronic device, or may be implemented by a system including the first electronic device and the second electronic device that is communicatively connected to the first electronic device. The method may include but is not limited to some or all of the following steps.

S601: The first electronic device receives a first instruction, and the first instruction indicates to periodically measure blood oxygen saturation.

In an implementation, the first electronic device may generate the first instruction in response to the user operation input by the user for the first control in the user interface shown in FIG. 3B.

In another implementation, the first electronic device may receive the first instruction sent by the second electronic device. The second electronic device may be a device (such as a mobile phone or a tablet computer) that establishes a communication connection to the first electronic device. The second electronic device may send the first instruction to the first electronic device in response to a user operation that is input by the user and that indicates to periodically measure the blood oxygen saturation.

Further, in response to the first instruction, the first electronic device may perform the following steps S602 to S606 or perform operations of S602 to S606 in each blood oxygen measurement period. The following uses a first blood oxygen measurement period as an example for description. Periodically measuring the blood oxygen saturation means that the blood oxygen saturation is measured at an interval of a measurement period T. Herein, the blood oxygen measurement period is a blood oxygen measurement process, and includes blood oxygen measurement time and non-blood oxygen measurement time. The blood oxygen measurement time is a time period in which the blood oxygen measurement is performed in the blood oxygen measurement period. The non-blood oxygen time is a time period other than the blood oxygen measurement time in the blood oxygen measurement period.

S602: The first electronic device determines, in the first blood oxygen measurement period, whether current time is the blood oxygen measurement time. If the current time is the blood oxygen measurement time, S603 is performed; otherwise, S602 is repeatedly performed.

The blood oxygen measurement time is the time that is determined by the periodic blood oxygen measurement method and that is required for performing the blood oxygen measurement. For example, if the blood oxygen measurement period T is 10 min, the blood oxygen measurement time for the first time is 20:00, the second time is 20:10, and the third time is 20:10.

Optionally, when theoretical start time of the blood oxygen measurement is reached, if the user is in a motion state, a time when the user changes to a static state is used as an actual start time of the blood oxygen measurement, and a next theoretical start time of the blood oxygen measurement is a time point obtained by adding the measurement period T to the current theoretical start time of the blood oxygen measurement or the current actual start time of the blood oxygen measurement. It should be understood that the first electronic device may analyze the motion state of the user based on motion data obtained by using an acceleration sensor, a gyroscope, or the like.

It should be understood that the first instruction may carry the blood oxygen measurement period T, to determine the blood oxygen measurement time based on the period. Alternatively, the first instruction may not carry the blood oxygen measurement period T, and the measurement period T may be preset duration.

It should be further understood that the blood oxygen measurement time is not one time point, but a plurality of periodic time points or time periods, and a period of the blood oxygen measurement time is the measurement period T.

S603: The first electronic device detects whether the user is in a sleep state at the current time. If yes, to be specific, the user is in the sleep state, the first electronic device may perform S604; otherwise, to be specific, the user is in a non-sleep state, the first electronic device performs S605.

Detection of the sleep state may include but is not limited to the following implementations:
Implementation 1: The first electronic device may detect a posture of the user by using a motion sensor. When the posture of the user continuously changes, it is determined that the user is in the non-sleep state. Conversely, when the posture of the user is static or unchanged, it is determined that the user is in the sleep state.
Implementation 2: The first electronic device may collect sound information of a current environment by using a microphone, extract a breathing sound in the sound information, and identify, based on the extracted breathing sound, whether the user is in the sleep state.
Implementation 3: The first electronic device may control a first LED to emit green light to the user, the green light emitted by the first LED is received by a first photoelectric detector after being reflected by the user, and the first photoelectric detector converts the received green light into a green light PPG signal. Further, the first electronic device may detect a heart rate and/or a heart rate-related parameter of the user based on the green light PPG signal, to detect the sleep state of the user based on the heart rate and/or the heart rate-related parameter of the user. It should be understood that, for a same person, the heart rate in the sleep state is lower than the heart rate in the non-sleep state.

In some embodiments, the first electronic device may alternatively control the first LED to emit red light or infrared light to the user, and correspondingly detect the heart rate and/or the heart rate-related parameter of the user based on a red light PPG signal or an infrared light PPG signal.

It should be noted that, with reference to the foregoing three implementations, the first electronic device may alternatively detect whether the user is in the sleep state, to obtain a more accurate detection result.

It should be further noted that the first electronic device may send, to the second electronic device, the posture detected by the motion sensor, the sound information collected by the microphone, the PPG signal collected by the first photoelectric detector, and the like; and the second electronic device detects whether the user is in the sleep state, and sends a detection result to the first electronic device.

S604: The first electronic device determines whether the sleep state of the user detected in a previous blood oxygen measurement period of the first blood oxygen measurement period is the non-sleep state. If yes, dimming needs to be performed, and the first electronic device may perform S605. Otherwise, dimming does not need to be performed, and the first electronic device may perform S606 and S607.

Specifically, when the sleep state detected in the previous blood oxygen measurement period of the first blood oxygen measurement period is the non-sleep state, and it is detected that the user is in the sleep state in the first blood oxygen measurement period, it indicates that the user changes from the non-sleep state to the sleep state. In this case, dimming needs to be performed. The first electronic device may perform S605, and perform S606 and S607 after dimming is completed. When the sleep state detected in the previous blood oxygen measurement period of the first blood oxygen measurement period is also the sleep state, it is determined that no change from the non-sleep state to the sleep state occurs. In this case, dimming does not need to be performed, and the first electronic device may perform S606 and S607.

S605: The first electronic device performs dimming.

Dimming is performed is to obtain a stable and effective PPG signal. In specific implementation, a light emitting power of an LED may be adjusted, so that an electrical signal obtained by converting light detected by a PD is greater than a specified PD target current, to ensure quality of the PPG signal. In an actual adjustment process, an LED drive current is adjusted to detect whether an output current of the PD reaches the target current. If the output current of the PD does not reach the target current, the LED drive current continues to be increased. If the output current of the PD reaches the target current, dimming ends. The entire dimming process is a negative feedback process.

Specifically, a light emitting power of an LED of an R path and a light emitting power of an LED of an IR path are adjusted to be respectively a first power and a second power. The first power is a transmit power of the LED of the R path applied when an output current of a PD of the R path is greater than or equal to a first target current. The second power is a transmit power of the LED of the IR path applied when an output current of a PD of the IR path is greater than or equal to a second target current.

It should be understood that the first target current and the second target current may be the same or different. The first target current of the user in the sleep state may be the same as or different from the first target current of the user in the non-sleep state, and the first target current of the user in the sleep state may be less than the first target current of the user in the non-sleep state. Similarly, the second target current of the user in the sleep state may be the same as or different from the second target current of the user in the non-sleep state, and the second target current of the user in the sleep state may be less than the second target current of the user in the non-sleep state.

In some embodiments, when the user is in the non-sleep state, and the current time is the blood oxygen measurement time, the first electronic device needs to perform dimming before obtaining the PPG signal, to obtain optimal data.

In some embodiments, when the user is in the non-sleep state, before S604, the first electronic device may obtain motion data of the user from previous dimming to the current time. When a motion amplitude of the first electronic device from the previous dimming to the current time is less than a preset value, the first electronic device may not perform dimming, but perform S605 and S606. Otherwise, the first electronic device performs S604, to perform dimming.

S606: The first electronic device collects the red light PPG signal and the infrared light PPG signal.

The first electronic device may turn on the R path and the IR path, to collect the red light PPG signal and the infrared light PPG signal. This specifically includes:

The first electronic device controls the LED of the R path to emit red light at the first power, and the LED of the IR path to emit infrared light at the second power. Further, the red light PPG signal and the infrared light PPG signal are respectively collected by using the PD of the R path and the PD of the IR path.

S607: The first electronic device calculates the SpO2 based on the red light PPG signal and the infrared light PPG signal.

Specifically, the first electronic device may separately collect a red light PPG signal with duration t1 and an infrared light PPG signal with duration t1. The duration t1 is greater than a heart rate period. Optionally, the duration t1 is greater than a plurality of heart rate periods, to improve SpO2 accuracy.

In some embodiments, after S607, the first electronic device may turn off the LED of the R path, the LED of the IR path, the PD of the R path, and the PD of the IR path at the non-blood oxygen measurement time, to further reduce energy consumption of the first electronic device.

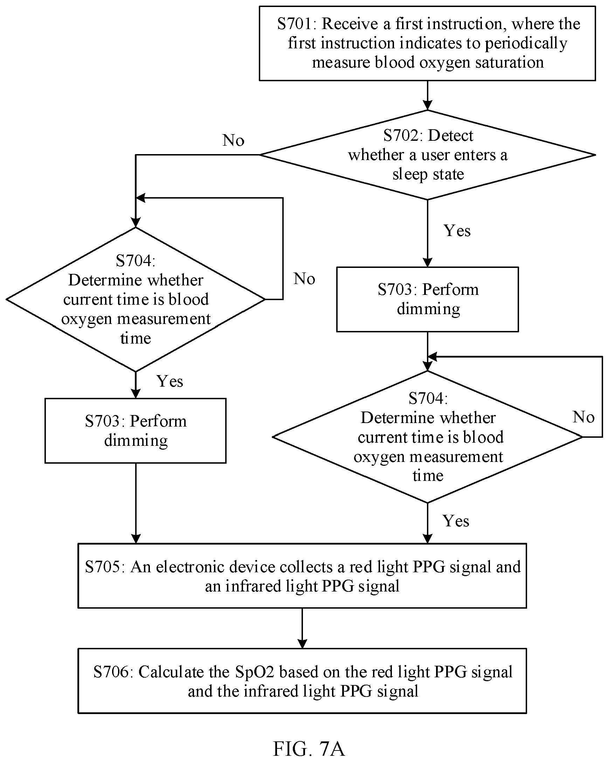

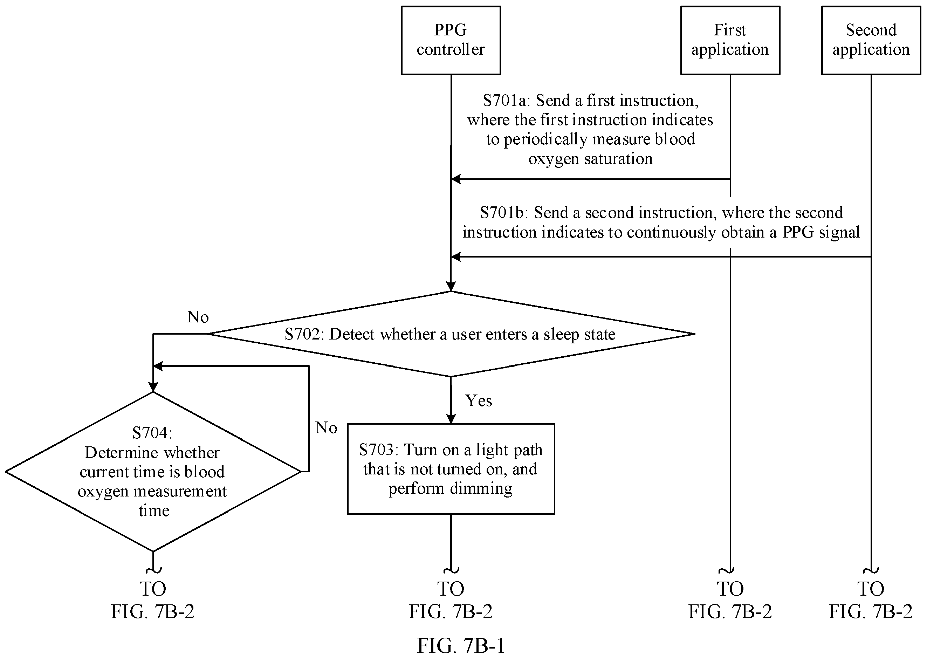

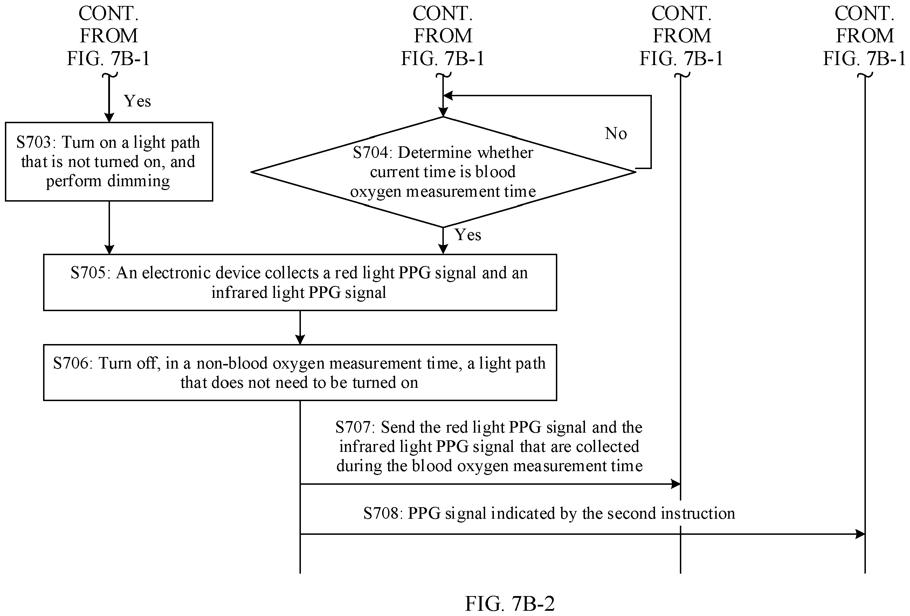

FIG. 7A is a schematic flowchart of another method for periodically measuring blood oxygen according to an embodiment of this application. The method may be independently implemented by the first electronic device, or may be implemented by a system including the first electronic device and the second electronic device that is communicatively connected to the first electronic device. The method may include but is not limited to some or all of the following steps.

S701: The first electronic device receives a first instruction, and the first instruction indicates to periodically measure blood oxygen saturation.

In response to the first instruction, the first electronic device may perform the following steps S702 to S705 or perform operations of S702 to S706 in each blood oxygen measurement period.

S702: The first electronic device detects whether the user enters a sleep state. If the user enters the sleep state, the first electronic device performs S703 and S704; otherwise, the first electronic device performs S704 and then performs S703.

That the first electronic device detects whether the user enters the sleep state means that the user changes from a non-sleep state to the sleep state. For specific implementation of detecting whether the user is in the sleep state, refer to related descriptions in the step S603 in the embodiment shown in FIG. 6. Details are not described herein again.

S703: The first electronic device performs dimming.

S704: The first electronic device determines whether current time is blood oxygen measurement time.

When a detection result of S702 is that the user enters the sleep state, if the first electronic device determines that the current time is the blood oxygen measurement time, the first electronic device may perform S705 and S706. When the detection result of S702 is that the user is in the non-sleep state, if the first electronic device determines that the current time is the blood oxygen measurement time, the first electronic device performs S703 to perform dimming, and after dimming is completed, S705 and S706 may be performed.

S705: The first electronic device collects a red light PPG signal and an infrared light PPG signal.

S706: The first electronic device calculates the SpO2 based on the red light PPG signal and the infrared light PPG signal.

For specific implementations of the steps S701 to S706, refer to related descriptions in the embodiment shown in FIG. 6. Details are not described herein again.

With reference to the method for periodically measuring blood oxygen shown in each of FIG. 6 and FIG. 7A, in some embodiments, the LED and the PD of the first electronic device each may be used as a blood oxygen sensor, or may be used as a heart rate sensor, or may be invoked by a plurality of applications. FIG. 7B-1 and FIG. 7B-2 are a schematic flowchart of another method for periodically measuring blood oxygen saturation according to an embodiment of this application. A first application and a second application may respectively send a first instruction and a second instruction to a PPG controller of the first electronic device, and the PPG controller may receive the second instruction. The second instruction indicates to continuously obtain a PPG signal. The PPG signals indicated by the second instruction may include: a red light PPG signal, an infrared light PPG signal, the red light PPG signal and the infrared light PPG signal, or other signals. After introducing the first instruction and the second instruction, the PPG of the first electronic device may perform the method shown in each of FIG. 6 and FIG. 7A, and may further turn on, in response to the second instruction, a light path corresponding to the second instruction. FIG. 7B-1 and FIG. 7B-2 are described by using an example in which the method shown in FIG. 7A is performed. Further, because one or more light paths are turned on in a previous blood oxygen measurement period, only a light path which is not turned on may be turned on before dimming. After PPG data required for a current blood oxygen measurement period is collected, the light path turned on before dimming may be turned off. Further, the red light PPG signal and the infrared light PPG signal that are required in the current blood oxygen measurement period may be sent to the first application, and the PPG signal indicated by the second instruction may be sent to the second application. For specific implementation of all steps in FIG. 7B-1 and FIG. 7B-2, refer to related descriptions in FIG. 4 and FIG. 7A. Details are not described herein again. Corresponding to the four cases of the PPG signal indicated by the second instruction, the R path or the IR path of the first electronic device may be set to or required to: continuously turn on an R path, continuously turn on an IR path, continuously turn on the R path and the IR path, or continuously turn on neither the R path nor the IR path. The four cases are separately described as follows.

When the first electronic device is set to continuously turn on the R path, for example, when the first electronic device is in a sleep mode of "scientific sleep", the first electronic device needs to continuously collect a red light PPG signal when the user is in a sleep state, to detect a sleep status of a user. That is, after detecting that the user enters the sleep state, the first electronic device needs to continuously turn on the R path. In this case, after S606 or S705 is performed, that is, after the PPG signal used for periodically measuring SpO2 is collected, the first electronic device enters non-blood oxygen measurement time. In this case, the first electronic device may keep the R path turned-on, turn off the IR path, to be specific, keep controlling an LED of the R path to emit red light at a first power and keep collecting the red light PPG signal by using a PD of the R path, and turn off an LED of the IR path and a PD of the IR path. However, during next blood oxygen measurement time, the first electronic device may turn on a path which is not turned on, that is, the IR path, and turn off the IR path after obtaining the infrared light PPG signal, to reduce energy consumption of the first electronic device.

When the first electronic device is set to continuously turn on the IR path, for example, when the first electronic device is in a sleep mode of "scientific sleep", the first electronic device needs to continuously collect the infrared light PPG signal when the user is in a sleep state, to detect a sleep status of the user based on the infrared light PPG signal. That is, after detecting that the user enters the sleep state, the first electronic device needs to continuously turn on the IR path. In this case, after S606 or S705 is performed, that is, after the PPG signal used for periodically measuring SpO2 is collected, the first electronic device enters non-blood oxygen measurement time. In this case, the first electronic device may keep the IR path turned-on, turn off the R path, to be specific, keep controlling an LED of the IR path to emit red light at a second power and keep collecting the infrared light PPG signal by using a PD of the IR path, and turn off an LED of the R path and a PD of the R path. However, during next blood oxygen measurement time, the first electronic device may turn on a path which is not turned on, that is, the R path, and turn off the R path after obtaining the red light PPG signal, to reduce energy consumption of the first electronic device.

When the first electronic device is set to continuously turn on the R path and the IR path, for example, when the first electronic device is in a sleep mode of "sleep apnea", the first electronic device needs to continuously collect the red light PPG signal and the infrared light PPG signal when the user is in a sleep state, to detect a sleep status of the user based on the red light PPG signal and the infrared light PPG signal. That is, after detecting that the user enters the sleep state, the first electronic device needs to continuously turn on the R path and the IR path. In this case, after S606 or S705 is performed, that is, after the PPG signal used for periodically measuring SpO2 is collected, the first electronic device enters non-blood oxygen measurement time. In this case, the first electronic device may keep the R path and the IR path turned-on, to be specific, keep controlling an LED of the R path to emit red light at a first power and keep collecting the red light PPG signal by using a PD of the R path, and keep controlling an LED of the IR path to emit red light at a second power and keep collecting the infrared light PPG signal by using a PD of the IR path. However, during next blood oxygen measurement time, the first electronic device may directly obtain the red light PPG signal and the infrared light PPG signal.

It should be noted that the blood oxygen measurement time is a time determined by the periodic blood oxygen measurement method. However, when the first electronic device is in the sleep mode of "sleep apnea", the first electronic device needs to monitor SpO2 in real time, and therefore the R path and the IR path need to be continuously turned on, to obtain the red light PPG signal and the infrared light PPG signal. However, a time period for collecting the PPG signal is not the blood oxygen measurement time specified in this application. The non-blood oxygen measurement time is a time other than the blood oxygen measurement time.

When the first electronic device is set to continuously turn on neither the R path nor the IR path, for example, when the first electronic device is in a sleep mode of "ordinary sleep", and when the user is in a non-sleep state or when current time is daytime, the first electronic device does not need to continuously collect the red light PPG signal and the infrared light PPG signal. In this case, after S606 or S705 is performed, that is, after the PPG signal used for periodically measuring SpO2 is collected, the first electronic device enters non-blood oxygen measurement time, and the first electronic device may turn off the R path and the IR path, to be specific, turn off an LED of the R path, an LED of the IR path, a PD of the R path, and a PD of the IR path, to reduce energy consumption of the first electronic device. However, during next blood oxygen measurement time, the first electronic device needs to turn on the R path and the IR path again.

FIG. 8A and FIG. 8B are a schematic flowchart of still another method for periodically measuring blood oxygen according to an embodiment of this application. The method may be implemented by the first electronic device, or may be implemented by a system including the first electronic device and the second electronic device that is communicatively connected to the first electronic device. The method may include but is not limited to some or all of the following steps.

S801: The first electronic device receives a first instruction, and the first instruction indicates to periodically measure blood oxygen saturation.

In each blood oxygen measurement period, the first electronic device may perform, in response to the first instruction, the following step S802 and a method procedure corresponding to a mode in Mode 1 to Mode 5.

S802: The first electronic device identifies a measurement mode based on a status of a user and settings of an R path and an IR path.

Specifically, the first electronic device may identify the status of the user, and the status of the user includes a sleep state and a non-sleep state. For specific implementation of identifying the sleep state of the user, refer to the step S603 in the embodiment shown in FIG. 6. Details are not described herein again.

Setting information of a PPG may include: continuously turning on neither the R path nor the IR path, continuously turning on the R path, continuously turning on the IR path, and continuously turning on the R path and the IR path. For example, four measurement modes shown in Table 1 may be obtained:

**Table 1**

| Measurement mode | Model 1 | Mode 2 | Mode 3 | Mode 4 | Mode 5 |
|---|---|---|---|---|---|
| Status of a user | Sleep state | Sleep state | Sleep state | Sleep state | Non-sleep state |
| Settings of an R path and an IR path | Continuously turn on neither the R path nor the IR path | Continuously turn on the R path | Continuously turn on the IR path | Continuously turn on the R path and the IR path | Continuously turn on neither the R path nor the IR path |

Further, the first electronic device may perform different method procedures for different measurement modes. Description is separately provided below.

### Model 1:

S803a: Determine whether current time is blood oxygen measurement time; if yes, perform S803b; otherwise, perform S802.

S803b: Determine whether the user is in the sleep state for the first time, and if yes, perform S803c, or if no, perform S803d to S803g. If the status of the user in a previous blood oxygen measurement period of a current blood oxygen measurement period includes the non-sleep state, it is determined that the user is in the sleep state for the first time.

S803c: The first electronic device turns on the R path and the IR path, to perform dimming.

S803d: The first electronic device collects a red light PPG signal and an infrared light PPG signal.

S803e: The first electronic device calculates SpO2 based on the red light PPG signal and the infrared light PPG signal.

S803f: The first electronic device turns off the R path and the IR path.

In another implementation, the first electronic device may alternatively first perform S803b; perform S803c when the user is in the sleep state for the first time, or perform S803a when the user is not in the sleep state for the first time; and perform S803d to S803f when the current time is the blood oxygen measurement time, or perform S802 when the current time is non-blood oxygen measurement time.

### Mode 2:

S804a: Determine whether current time is blood oxygen measurement time; if yes, perform S804b; otherwise, perform S802.

S804b: Determine whether the user is in the sleep state for the first time, and if yes, perform S804c, or if no, perform S804d to S804f.

S804c: The first electronic device turns on the IR path, to perform dimming.

S804d: The first electronic device collects a red light PPG signal and an infrared light PPG signal.

S804e: The first electronic device calculates SpO2 based on the red light PPG signal and the infrared light PPG signal.

S804f: The first electronic device turns off the IR path.

In another implementation, the first electronic device may alternatively first perform S804b; perform S804c when the user is in the sleep state for the first time, or perform S804a when the user is not in the sleep state for the first time; and perform S804d to S804f when the current time is the blood oxygen measurement time, or perform S802 when the current time is non-blood oxygen measurement time.

### Mode 3:

S805a: Determine whether current time is blood oxygen measurement time; if yes, perform S805b; otherwise, perform S802.

S805b: Determine whether the user is in the sleep state for the first time, and if yes, perform S805c, or if no, perform S805d to S805f.

S805c: The first electronic device turns on the R path, to perform dimming.

S805d: The first electronic device collects a red light PPG signal and an infrared light PPG signal.

S805e: The first electronic device calculates SpO2 based on the red light PPG signal and the infrared light PPG signal.

S805f: The first electronic device turns off the R path.

In another implementation, the first electronic device may alternatively first perform S805b; perform S805c when the user is in the sleep state for the first time, or perform S805a when the user is not in the sleep state for the first time; and perform S805d to S805ef when the current time is the blood oxygen measurement time, or perform S802 when the current time is non-blood oxygen measurement time.

### Mode 4:

S806a: Determine whether current time is blood oxygen measurement time; if yes, perform S806b; otherwise, perform S802.

S806b: Determine whether the user is in the sleep state for the first time, and if yes, perform S806c, or if no, perform S805d to S805f.

S806c: The first electronic device performs dimming.

S806d: The first electronic device collects a red light PPG signal and an infrared light PPG signal.

S806e: The first electronic device calculates SpO2 based on the red light PPG signal and the infrared light PPG signal.

In another implementation, the first electronic device may alternatively first perform S806b; perform S806c when the user is in the sleep state for the first time, or perform S806a when the user is not in the sleep state for the first time; and perform S806d and S806e when the current time is the blood oxygen measurement time, or perform S802 when the current time is non-blood oxygen measurement time.

### Mode 5:

S807a: Determine whether current time is blood oxygen measurement time; if yes, perform S805b to S805f; otherwise, perform S802.

S807b: The first electronic device turns on the R path and the IR path, to perform dimming.

S807c: The first electronic device collects a red light PPG signal and an infrared light PPG signal.

S807d: The first electronic device calculates the SpO2 based on the red light PPG signal and the infrared light PPG signal.

S807e: The first electronic device turns off the R path and the IR path.

In another implementation, before S805b, the first electronic device may obtain motion data of the user from previous dimming to the current time. When a motion amplitude of the first electronic device from the previous dimming to the current time is less than a preset value, the first electronic device may not perform dimming, but perform S805c to S805e.

In embodiments shown in FIG. 6 to FIG. 8A and FIG. 8B, the first electronic device may alternatively send the collected red light PPG signal and the collected infrared light PPG signal to the second electronic device, and the second electronic device calculates the SpO2 based on the red light PPG signal and the infrared light PPG signal.

In embodiments shown in FIG. 6 to FIG. 8A and FIG. 8B, a blood oxygen measurement period may be fixed, or may be dynamically set. For example, a period in which the user is in the sleep state may be greater than a period in which the user is in the non-sleep state, and a period in which the user is in a static state may be greater than a period in which the user is in a motion state.

Optionally, sampling frequencies of the PPG signal may be different for different sleep states, motion states, or measurement modes of different users. For example, a sampling frequency of a photoelectric detector of the R path applied when the user is in the sleep state is less than a sampling frequency of the photoelectric detector of the R path applied when the user is in the non-sleep state. Similarly, a sampling frequency of a photoelectric detector of the IR path applied when the user is in the sleep state may be less than a sampling frequency of the photoelectric detector of the IR path applied when the user is in the non-sleep state. For another example, a sampling frequency when the user is in the static state may be greater than a sampling frequency when the user is in the motion state. For another example, a sampling frequency when a test mode is Mode 1, Mode 2, Mode 3, or Mode 4 is greater than a sampling frequency when the test mode is Mode 5.

In embodiments shown in FIG. 6 to FIG. 8A and FIG. 8B, the method may further include: outputting first information when the SpO2 calculated by the first electronic device or the second electronic device is less than a first threshold, where the first information is used to prompt the user that there is a risk of sleep apnea; or outputting second information when the SpO2 calculated by the electronic device in a first time period (for example, one or more days) is less than a second threshold or a probability that the SpO2 is less than the second threshold is greater than a third threshold, where the second information outputs prompt information used to prompt the user that there is a risk of sleep apnea, and is used to prompt the user to seek medical treatment.

The foregoing descriptions are merely specific implementations of this application, but are not intended to limit the protection scope of this application. Any variation or replacement within the technical scope disclosed in this application shall fall within the protection scope of this application. Therefore, the protection scope of this application shall be subject to the protection scope of the claims.

## Claims

1. A method for periodically measuring blood oxygen saturation, applied to an electronic device, wherein the electronic device comprises a red light R path and an infrared light IR path, the R path and the IR path each comprise a light emitting source and a photoelectric detector, the light emitting source of the R path is configured to emit red light to a user, the photoelectric detector of the R path is configured to convert red light reflected by the user into a red light photoplethysmography PPG signal, the light emitting source of the IR path is configured to emit infrared light to the user, and the photoelectric detector of the IR path is configured to convert infrared light reflected by the user into an infrared light photoplethysmography PPG signal; and
the method comprises:
receiving, by the electronic device, a first instruction, wherein the first instruction indicates to periodically measure blood oxygen saturation;
performing, by the electronic device in response to the first instruction, dimming in a first blood oxygen measurement period if the user changes from a non-sleep state to a sleep state, wherein the dimming is used to determine that a light emitting power of the light emitting source of the R path and a light emitting power of the light emitting source of the IR path are respectively a first power and a second power;
during blood oxygen measurement time in a second blood oxygen measurement period, if the user is in the sleep state, controlling, by the electronic device, the light emitting source of the R path to emit the red light at the first power and the light emitting source of the IR path to emit the infrared light at the second power, wherein the second blood oxygen measurement period and the first blood oxygen measurement period are a same blood oxygen measurement period, or the second period is a next blood oxygen measurement period of the first blood oxygen measurement period; and
respectively collecting the red light PPG signal and the infrared light PPG signal by using the photoelectric detector of the R path and the photoelectric detector of the IR path, wherein the red light PPG signal and the infrared light PPG signal are used to calculate the blood oxygen saturation.

2. The method according to claim 1, wherein the second blood oxygen measurement period and the first blood oxygen measurement period are the same blood oxygen measurement period, and the performing dimming in a first blood oxygen measurement period if the user changes from a non-sleep state to a sleep state comprises:
detecting, by the electronic device, a current sleep status of the user when current time is the blood oxygen measurement time; and
when the current sleep status is the sleep state and the user is in the non-sleep state in a previous blood oxygen measurement period of the first blood oxygen measurement period, performing, by the electronic device, dimming.

3. The method according to claim 1 or 2, wherein the method further comprises:
turning off the light emitting source of the R path, the light emitting source of the IR path, the photoelectric detector of the R path, and the photoelectric detector of the IR path during non-blood oxygen measurement time, wherein the non-blood oxygen measurement time is a time period other than the blood oxygen measurement time in the blood oxygen measurement period.

4. The method according to claim 1 or 2, wherein before the controlling the light emitting source of the R path to emit the red light at the first power and the light emitting source of the IR path to emit the infrared light at the second power, the method further comprises:
receiving, by the electronic device, a second instruction, wherein the second instruction indicates to continuously obtain a PPG signal.

5. The method according to claim 4, wherein the electronic device further comprises a PPG manager, and the PPG manager of the electronic device is configured to perform the following steps:
generating a control signal based on the first instruction and the second instruction, wherein the first instruction comes from a first application, the second instruction comes from a second application, and the control signal is used to drive the light emitting source of the R path to emit the red light at the first power and the light emitting source of the IR path to emit the infrared light at the second power; and
sending the red light PPG signal and the infrared light PPG signal to the first application, and sending, to the second application, the PPG signal obtained based on the indication of the second instruction.

6. The method according to claim 4, wherein when the PPG signal obtained based on the indication of the second instruction is the red light PPG signal, the method further comprises:
during non-blood oxygen measurement time, keeping controlling the light emitting source of the R path to emit the red light at the first power and keeping collecting the red light PPG signal by using the photoelectric detector of the R path, wherein the non-blood oxygen measurement time is a time period other than the blood oxygen measurement time in the second blood oxygen measurement period; and
turning off the light emitting source of the IR path and the photoelectric detector of the IR path during the non-blood oxygen measurement time.

7. The method according to claim 4, wherein when the PPG signal obtained based on the indication of the second instruction is the infrared light PPG signal, the method further comprises:
during non-blood oxygen measurement time, keeping controlling the light emitting source of the IR path to emit the red light at the second power and keeping collecting the infrared light PPG signal by using the photoelectric detector of the IR path, wherein the non-blood oxygen measurement time is a time period other than the blood oxygen measurement time in the second blood oxygen measurement period; and
turning off the light emitting source of the R path and the photoelectric detector of the R path during the non-blood oxygen measurement time.

8. The method according to claim 4, wherein when the PPG signal obtained based on the indication of the second instruction is the red light PPG signal and the infrared light PPG signal, the method further comprises:
during non-blood oxygen measurement time, keeping controlling the light emitting source of the R path to emit the red light at the first power, keeping controlling the light emitting source of the IR path to emit the infrared light at the second power, keeping collecting the red light PPG signal by using the photoelectric detector of the R path, and keeping collecting the infrared light PPG signal by using the photoelectric detector of the IR path, wherein the non-blood oxygen measurement time is a time period other than the blood oxygen measurement time in the second blood oxygen measurement period.

9. The method according to any one of claims 1 to 8, wherein the method further comprises:
performing, by the electronic device, dimming when the user is in the non-sleep state during the blood oxygen measurement time, wherein the dimming is used to determine that the light emitting power of the light emitting source of the R path and the light emitting power of the light emitting source of the IR path are respectively the first power and the second power

10. The method according to claim 9, wherein the performing, by the electronic device, dimming when the user is in the non-sleep state during the blood oxygen measurement time specifically comprises:
when the user is in the non-sleep state during the blood oxygen measurement time, obtaining, by the electronic device, motion data of the user from time of previous dimming to current time; and
when it is identified, based on the motion data, that a motion amplitude of the user from the previous dimming to the current time is less than a preset value, adjusting, by the electronic device, the light emitting power of the light emitting source of the R path and the light emitting power of the light emitting source of the IR path to be respectively the first power and the second power

11. The method according to any one of claims 1 to 10, wherein the first power is a transmit power of the light emitting source of the R path applied when an output current of the photoelectric detector of the R path is greater than or equal to a first target current; and the second power is a transmit power of the light emitting source of the IR path applied when an output current of the photoelectric detector of the IR path is greater than or equal to a second target current.

12. The method according to any one of claims 1 to 11, wherein a sampling frequency of the photoelectric detector of the R path applied when the user is in the sleep state is less than a sampling frequency of the photoelectric detector of the R path applied when the user is in the non-sleep state; and a sampling frequency of the photoelectric detector of the IR path applied when the user is in the sleep state is less than a sampling frequency of the photoelectric detector of the IR path applied when the user is in the non-sleep state.

13. The method according to any one of claims 1 to 12, wherein the light emitting source of the R path and the light emitting source of the IR path are a same light emitting source, and the photoelectric detector of the R path and the photoelectric detector of the IR path are a same photoelectric detector

14. An electronic device, wherein the electronic device comprises a photoplethysmography PPG controller, a memory, a red light R path, and an infrared light IR path, the R path and the IR path each comprise a light emitting source and a photoelectric detector, the light emitting source of the R path is configured to emit red light to a user, the photoelectric detector of the R path is configured to convert red light reflected by the user into a red light photoplethysmography PPG signal, the light emitting source of the IR path is configured to emit infrared light to the user, and the photoelectric detector of the IR path is configured to convert infrared light reflected by the user into an infrared light photoplethysmography PPG signal; and
the PPG controller runs computer instructions stored in the memory, to perform the following steps:
receiving a first instruction, wherein the first instruction indicates to periodically measure blood oxygen saturation;
performing, in response to the first instruction, dimming in a first blood oxygen measurement period if the user changes from a non-sleep state to a sleep state, wherein the dimming is used to determine that a light emitting power of the light emitting source of the R path and a light emitting power of the light emitting source of the IR path are respectively a first power and a second power;
during blood oxygen measurement time in a second blood oxygen measurement period, if the user is in the sleep state, controlling the light emitting source of the R path to emit the red light at the first power and the light emitting source of the IR path to emit the infrared light at the second power, wherein the second blood oxygen measurement period and the first blood oxygen measurement period are a same blood oxygen measurement period, or the second period is a next blood oxygen measurement period of the first blood oxygen measurement period; and
respectively collecting the red light PPG signal and the infrared light PPG signal by using the photoelectric detector of the R path and the photoelectric detector of the IR path, wherein the red light PPG signal and the infrared light PPG signal are used to calculate the blood oxygen saturation.

15. The electronic device according to claim 14, wherein the second blood oxygen measurement period and the first blood oxygen measurement period are the same blood oxygen measurement period, and that the PPG controller performs dimming in a first blood oxygen measurement period if the user changes from a non-sleep state to a sleep state comprises:
detecting a current sleep status of the user when current time is the blood oxygen measurement time; and
when the current sleep status is the sleep state and the user is in the non-sleep state in a previous blood oxygen measurement period of the first blood oxygen measurement period, performing dimming.

16. The electronic device according to claim 14 or 15, wherein the PPG controller is further configured to:
turn off the light emitting source of the R path, the light emitting source of the IR path, the photoelectric detector of the R path, and the photoelectric detector of the IR path during non-blood oxygen measurement time, wherein the non-blood oxygen measurement time is a time period other than the blood oxygen measurement time in the blood oxygen measurement period.

17. The electronic device according to claim 14 or 15, wherein before the PPG manager controls the light emitting source of the R path to emit the red light at the first power and the light emitting source of the IR path to emit the infrared light at the second power, the PPG controller is further configured to:
receive a second instruction, wherein the second instruction indicates to continuously obtain a PPG signal.

18. The electronic device according to claim 17, wherein the PPG manager is configured to perform the following steps:
generating a control signal based on the first instruction and the second instruction, wherein the first instruction comes from a first application, the second instruction comes from a second application, and the control signal is used to drive the light emitting source of the R path to emit the red light at the first power and the light emitting source of the IR path to emit the infrared light at the second power; and
sending the red light PPG signal and the infrared light PPG signal to the first application, and sending, to the second application, the PPG signal obtained based on the indication of the second instruction.

19. The electronic device according to claim 17, wherein when the PPG signal obtained based on the indication of the second instruction is the red light PPG signal, the PPG manager is further configured to:
during non-blood oxygen measurement time, keep controlling the light emitting source of the R path to emit the red light at the first power and keep collecting the red light PPG signal by using the photoelectric detector of the R path, wherein the non-blood oxygen measurement time is a time period other than the blood oxygen measurement time in the second blood oxygen measurement period; and
turn off the light emitting source of the IR path and the photoelectric detector of the IR path during the non-blood oxygen measurement time.

20. The electronic device according to claim 17, wherein when the PPG signal obtained based on the indication of the second instruction is the infrared light PPG signal, the PPG manager is further configured to:
during non-blood oxygen measurement time, keep controlling the light emitting source of the IR path to emit the red light at the second power and keep collecting the infrared light PPG signal by using the photoelectric detector of the IR path, wherein the non-blood oxygen measurement time is a time period other than the blood oxygen measurement time in the second blood oxygen measurement period; and
turn off the light emitting source of the R path and the photoelectric detector of the R path during the non-blood oxygen measurement time.

21. The electronic device according to claim 17, wherein when the PPG signal obtained based on the indication of the second instruction is the red light PPG signal and the infrared light PPG signal, the PPG manager is further configured to:
during non-blood oxygen measurement time, keep controlling the light emitting source of the R path to emit the red light at the first power, keep controlling the light emitting source of the IR path to emit the infrared light at the second power, keep collecting the red light PPG signal by using the photoelectric detector of the R path, and keep collecting the infrared light PPG signal by using the photoelectric detector of the IR path, wherein the non-blood oxygen measurement time is a time period other than the blood oxygen measurement time in the second blood oxygen measurement period.

22. The electronic device according to any one of claims 14 to 21, wherein the PPG manager is further configured to:
perform dimming when the user is in the non-sleep state during the blood oxygen measurement time, wherein the dimming is used to determine that the light emitting power of the light emitting source of the R path and the light emitting power of the light emitting source of the IR path are respectively the first power and the second power.

23. The electronic device according to claim 22, wherein that the PPG manager performs dimming when the user is in the non-sleep state during the blood oxygen measurement time specifically comprises:
when the user is in the non-sleep state during the blood oxygen measurement time, obtaining motion data of the user from previous dimming to current time; and
when it is identified, based on the motion data, that a motion amplitude of the user from the previous dimming to the current time is less than a preset value, adjusting the light emitting power of the light emitting source of the R path and the light emitting power of the light emitting source of the IR path to be respectively the first power and the second power.

24. The electronic device according to any one of claims 14 to 23, wherein the first power is a transmit power of the light emitting source of the R path applied when an output current of the photoelectric detector of the R path is greater than or equal to a first target current; and the second power is a transmit power of the light emitting source of the IR path applied when an output current of the photoelectric detector of the IR path is greater than or equal to a second target current.

25. The electronic device according to any one of claims 14 to 24, wherein a sampling frequency of the photoelectric detector of the R path applied when the user is in the sleep state is less than a sampling frequency of the photoelectric detector of the R path applied when the user is in the non-sleep state; and a sampling frequency of the photoelectric detector of the IR path when the user is in the sleep state is less than a sampling frequency of the photoelectric detector of the IR path when the user is in the non-sleep state.

26. The method according to any one of claims 14 to 25, wherein the light emitting source of the R path and the light emitting source of the IR path are a same light emitting source, and the photoelectric detector of the R path and the photoelectric detector of the IR path are a same photoelectric detector

27. A computer program product comprising instructions, wherein when the computer program product is run on an electronic device, the electronic device is enabled to perform the method according to any one of claims 1 to 14.

28. A computer-readable storage medium, comprising instructions, wherein when the instructions are run on an electronic device, the electronic device is enabled to perform the method according to any one of claims 1 to 14.
